(19) Europäisches Patentamt

European Patent Office

Office européen des brevets

(11) Veröffentlichungsnummer: **0 278 112 B1**

(12) **EUROPÄISCHE PATENTSCHRIFT**

(45) Veröffentlichungstag der Patentschrift: **18.11.93**

(21) Anmeldenummer: **87119221.7**

(22) Anmeldetag: **24.12.87**

(51) Int. Cl.5: **C07K 7/10, A61K 37/64, C12N 15/00, C12N 1/20**

---

(54) **Gentechnologisch hergestellter pankreatischer sekretorischer Trypsininhibitor und seine Varianten.**

---

(30) Priorität: **07.01.87 GB 8700204**

(43) Veröffentlichungstag der Anmeldung:
**17.08.88 Patentblatt 88/33**

(45) Bekanntmachung des Hinweises auf die
Patenterteilung:
**18.11.93 Patentblatt 93/46**

(84) Benannte Vertragsstaaten:
**AT BE CH DE ES FR GB GR IT LI NL SE**

(56) Entgegenhaltungen:
EP-A- 0 132 732    EP-A- 0 171 024
EP-A- 0 264 118    EP-A- 0 267 692
WO-A-86/03519      WO-A-88/03171

**J. BIOCHEMISTRY, Band 98, Nr. 3, Juni 1985; N. KIKUCHI et al., Seiten 687-694\***

**BIOCHEMICAL AND BIOPHYSICAL RESE-ARCH COMMUNICATIONS, Band 132, Nr. 2, 30 Oktober 1985, Academic Press, Inc.; T. YAMAMOTO et al.: Seiten 605-612\***

(73) Patentinhaber: **BAYER AG**

**D-51368 Leverkusen(DE)**

(72) Erfinder: **Collins, John, Prof. Dr.**
**Aegidienstrasse 5**
**D-3300 Braunschweig(DE)**
Erfinder: **Blöcker, Helmut, Dr.**
**Maschplatz 13**
**D-3300 Braunschweig(DE)**
Erfinder: **Frank, Ronald, Dr.**
**Leibnitzstrasse 8**
**D-3340 Wolfenbuettel(DE)**
Erfinder: **Maywald, Friedhelm, Dr.**
**Elzweg 32**
**D-3300 Braunschweig(DE)**
Erfinder: **Fritz, Hans, Prof. Dr.**
**Neulingerstrasse 15**
**D-8011 Hohenbrunn(DE)**
Erfinder: **Bruns, Wolfgang, Dr.**
**Kaiser Wilhelm-Allee 37**
**D-5600 Wuppertal 1(DE)**

---

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ARCHIVES OF BIOCHEMISTRY AND BIOPHY-
SICS, Band 179, 1977, Academic Press, Inc.,
New York, NY (US); D.C. BARTELT et al.,
Seiten 189-199*

THE EMBO JOURNAL, Band 5, Nr. 12, 1986,
IRL PressLtd., Oxford (GB); B. VON
WILCKEN-BERGMANN et al., Seiten
3219-3225*

JOURNAL OF BIOLOGICAL CHEMISTRY,
Band 261, Nr. 16, 05 Juni 1986, The American
Society of Biological Chemists, Inc. (US);
C.B. MARKSet al., Seiten 7115-7118*

CHEMICAL ABSTRACTS, Band 96, 1982, Columbus, OH (US); M. LASKOWSKI Jr. et al.,
Seite 338, Nr. 158093z*

CHEMICAL ABSTRACTS, Band 100, 1984, Columbus, OH (US); M. LASKOWSKI Jr. et al.,
Seite 241, Nr. 116990k*

BIOLOGICAL ABSTRACTS, RRM 34:19104,
Philadelphia, PA (US); M. SZARDENINGS et
al., Nr. AN 88:42084*

**Beschreibung**

Die vorliegende Erfindung betrifft ein mikrobiell hergestelltes Peptid, welches im wesentlichen die Aminosäuresequenz von humanem pankreatischen sekretorischen Trypsininhibitor (h-PSTI) aufweist. Die Erfindung betrifft im weiteren Varianten eines solchen Peptides, wobei eine oder mehrere der Aminosäuren in der ursprünglichen Sequenz durch andere Aminosäuren ersetzt werden. Diese Peptide weisen in bezug auf ihre Inhibitorwirkung eine vorteilhaft modifizierte Spezifität auf. Außerdem werden ein Herstellungsverfahren für die Peptide sowie deren pharmazeutische Verwendung beschrieben.

EINFÜHRUNG

Lysosomale Elastase (Leukocyten-Elastase); J. G. Bieth (1986), S. 217-320 (in Regulation of Matrix Accumulation, Mecham, Herausgeber, Academic Press, Orlando) aus polymorphkernigen Granulocyten stellt eine potente intrazelluläre Protease dar, die in Lysosomen gelagert wird, und ihre physiologische Funktion, den intrazellulären Proteinabbau, in Phagolysosomen bewerkstelligt. Die wichtigste funktionelle Rolle lysosomaler Proteasen (Elastase, Cathepsin G etc.; H. Fritz et al (1984) in Selected Topics in Clinical Enzymology, Goldberg und Werner Hsg., Walter de Gruyter, Berlin, Bd. 2, S. 305-328) ist der Abbau von phagocytiertem Material aus dem Organismus selbst (z.B. Stoffwechselprodukte, verletztes Gewebe) oder von invasiven Organismen (Bakterien, Viren, Schimmelpilzen etc.).

Nach Freisetzung in den extrazellularen Raum (Blut oder Interstitialflüssigkeit) wird Elastase rasch von wirksamen endogenen Inhibitoren, wie $\alpha_1$-PI ($\alpha_1$-Proteaseinhibitor;J. Travis und G. S. Salvesen (1983) Ann. Rev. Biochem., S. 655-709) im Plasma, und/oder Antileukoprotease (auch als HUSI-I, humaner seminaler Proteinaseinhibitor bezeichnet; H. Schiessler et al (1978), S. 195-207, in Neutral Proteases of Human Polymorphonuclear Leukocytes, Havemann und Janoff Hsg., Urban & Schwarzenberg, Baltimore) in Schleimabsonderungen, gebunden.

Im Falle eines erblichen $\alpha_1$-PI-Mangels (J. G. Bieth, 1986) oder als Folge einer massiven extrazellularen Elastasefreisetzung (bei akuten und chronischen Entzündungen, Polytrauma oder Schock; H. Fritz et al, 1984) ist der Schutz des Organismus gegenüber dem degradativen Elastasepotential durch natürliche Proteaseinhibitoren unzureichend. Ein exzessiver und lokal sogar vollständiger Verbrauch von endogenen Proteaseinhibitoren wird verursacht durch (i) Komplexbildung mit Elastase, (ii) proteolytische Inaktivierung durch verschiedene lysosomale Proteasen, und (iii) insbesondere durch oxidative Inaktivierung ($\alpha_1$-PI) (J. G. Bieth (1986); H. Fritz et al (1984); J. Travis und G. S. Salvesen (1983); siehe oben).

Die Folge davon ist ein extensiver proteolytischer Abbau von Bindegeweben sowie von humoralen Proteinen, einschließlich Koagulations-, Fibrinolyse- und Komplementfaktoren durch Elastase und andere lysosomale Proteasen (z. B. Cathepsin G), was zu ernsthaften klinischen Symptomen führt, wie Emphysem, Schocklunge, erworbenes respiratorisches Distress-Syndrom (ARDS), Koagulationsstörungen, Nieren- und Leberversagen etc. (zusätzlich zu den vorstehend genannten Referenzen: Neue Wege in der Entzündungsdiagnostik, PMN Elastase; M. Jochum et al (Hsg.), (1985) GIT Verlag, Darmstadt; C. T. Lee et al (1981) N. Engl. J. Med. 304, 192-196; W. W. McGuire et al (1982) J. Clin, Invest. 69, 543).

Elastase trägt auch zur lokalen Entzündung bei, wie sie bei rheumatoider Arthritis auftritt, z. B. dem Abbau von Bindegewebsbestandteilen (K. Kleesiek et al (1985) in: Neue Wege in der Entzündungsdiagnostik, PMN Elastase, S. 71-82).

Die extrazelluläre Freisetzung von Elastase nach schweren Verletzungen oder bei Erkrankungen, wie septischem Schock, Schocklunge etc., kann routinemäßig mit Hilfe von Enzymimmunoassays verfolgt werden (S. Neumann und M. Jochum (1984), S. 184-195 in Methods of Enzymatic Analysis, Bergmeyer (Hsg.), Verlag Chemie, Weinheim).

Bei experimentellen Modellen für Sepsis und Emphysem haben sich synthetische Elastaseinhibitoren (J. C. Powers Am. Rev. Respir. Dis. (1983) 127, 554-558) und natürliche Inhibitoren aus Tieren, wie Eglin C (H. P. Schnebli et al (1985), Eur. J. Respir. Dis. 66, Ergänzung 139, S. 66-70) als therapeutisch nützlich erwiesen. Die Anwendung eines Proteaseinhibitors menschlichen Ursprungs wäre vorzuziehen, um toxische Nebeneffekte und insbesondere allergische Reaktionen zu vermeiden, wenn eine Langzeit-Therapie indiziert ist, z. B. bei der Behandlung von $\alpha_1$-PI-Mangel (Emphysem). Da human-$\alpha_1$-PI ein Glykoprotein mit hohem Molekulargewicht darstellt, ist dessen Herstellung mit Hilfe der Gentechnologie in ausreichenden Mengen in naher Zukunft nicht zu erwarten.

Die Antileukoprotease oder HUSI-I (H. Schiessler et al, 1978; und U. Seemüller et al (1986), FEBS Letters 199, 43-48) hat ein MG von 14,000 Dalton und besteht aus zwei aktiven Domänen, wobei eine gegen Elastase, die andere gegen Trypsin gerichtet ist. Aus diesem Grunde hat dieser Inhibitortyp eine relative niedrige Selektivität und stellt nicht einen spezifischen Elastaseinhibitor dar. Die Inhibierung von Trypsin oder Trypsin-ähnlichen Enzymen ist üblicherweise bei den vorstehend gegebenen Indikationen nicht beabsichtigt.

Der menschliche Pankreas sezerniert PSTI, einen Proteaseinhibitor von niedrigem Molekulargewicht (6, 2 kd), der spezifisch Trypsin inhibiert, d. h. er besitzt eine hohe Selektivität für einen spezifischen Proteasetyp.

Ein Vorteil der vorliegenden Erfindung besteht in der Substitution von nur einer (oder einiger weniger) Aminosäuren in PSTI mit Hilfe der rekombinanten DNA-Technologie, wobei PSTI-Varianten erhalten werden, von denen gezeigt wurde, daß sie hochwirksame Proteaseinhibitoren mit einer hohen Spezifität für Leukocyten-Elastase darstellen. Außerdem sollte der Elastase-PSTI-Drivat-Komplex aufgrund seines relativ niedrigen Molekulargewichtes auch die Nieren passieren. Aus diesem Grunde wird die Eliminierung von extrazellulär freigesetzter Elastase sehr wirksam sein. Die Tatsache, daß PSTI menschlichen Ursprungs ist, zusammen mit dessen niedrigem Molekulargewicht, ließ uns erwarten, daß bei der klinischen Anwendung von PSTI-Derivaten keine Komplikationen auftreten, die auf die Erkennung dieser Substanzen als Fremdproteine durch das Immunsystem zurückgehen.

Ein weiterer wesentlicher Vorteil von PSTI, im Vergleich zu $\alpha_1$-PI und Antileukoprotease, liegt in der Unempfindlichkeit gegenüber oxidativer Inaktivierung während Entzündungsprozessen, bei denen stark oxidative Agenzien produziert und extrazellulär freigesetzt werden. Folglich sollten im Vergleich zu $\alpha_1$-PI und Antileukoprotease niedrigere Dosen des PSTI-Derivates ausreichend sein, um die gleiche Schutzwirkung zu erzielen.

Es ist somit Aufgabe der vorliegenden Erfindung, pharmazeutisch nützliche Peptide zur Verfügung zu stellen, die über eine Proteinaseinhibitorwirkung verfügen, vorzugsweise mit einer verbesserten Spezifität und/oder verbesserten Inhibitorwirkung. Die genannten Peptide stellen Peptide mit der Sequenz von PSTI und Varianten desselben dar, die durch rekombinante DNA-Technologie hergestellt werden. Die Bezeichnung "Varianten" bezieht sich auf Peptide, in denen eine oder mehrere Aminosäuren der Stammsequenz durch andere, natürlich vorkommende, Aminosäuren ersetzt sind. Als Stammsequenz dient die Sequenz des humanen PSTI (h-PSTI = PSTI O). Bevorzugte Positionen, die einem Austausch von Aminosäuren unterliegen, sind die Positionen 17, 18, 19, 20, 32 und 36 im Peptid. Die vorliegende Erfindung betrifft besonders bevorzugt Peptide, die im wesentlichen die Sequenz von PSTI O (wie sie von L. J. Greene, 1976, Methods Enzymol. 45, 813-825, beschrieben wird) aufweisen und die folgenden Aminosäuren umfassen:

Glu, Asp oder Leu in Position 13
Glu, Asp oder Asn in Position 14
Thr, Pro, Ser, Arg, Leu oder Met in Position 17;
Leu, Met, Val, Gln, Ser, Ala, Thr, Ile, Tyr, Phe, Arg, Trp oder Lys in Position 18;
Glu, Asp, Leu oder Ile in Position 19;
Tyr, Phe, Asp, Asn, Leu, Glu, Gln, His oder Arg in Position 20;
Glu, Arg, Met, Phe, Lys, Val, Thr, Gln, Leu, Asp oder Asn in Position 21;
Lys, Glu, Ile, Gln, Asp oder Asn in Position 29;
Pro, Gly, Ser, Asn, Ala, Asp, Val, Arg oder His in Position 32 und
Asn, Asp, Ala, Ser, Gly, Tyr, Val oder Glu in Position 36.

Ausgenommen sind die Kombinationen -Leu[13], - Asn[14], -Thr[17], - Lys[8], - Ile[19], - Tyr[20], - Asn[21], - Asp[29], - Pro[32], - Val[36] sowie - Leu[13], - Asn[14], - Ther[17], - Lys[18], - Ile[19], - Tyr[20], - Asp[21], - Asn[29], - Pro[32], - Val[36].

Die in Tabelle 1 dargestellten Varianten können im weiteren in Position 32 und 36 durch die vorstehend aufgeführten Aminosäuren variieren.

Die vorliegende Erfindung betrifft auch Peptide mit den vorstehend beschriebenen Sequenzen, die außerdem in Position 1 ein Methionin oder ein Leaderpeptid besitzen. Die Bezeichnung "Leaderpeptid" bedeutet in bezug auf die vorliegende Erfindung nicht nur Signalsequenzen, welche die Sekretion des Expressionsproduktes fördern (S. D. Emr et al, J. of Cell Biol., 1980, S. 701-711), sondern auch Peptide, welche eine Signalsequenz und eine Linkersequenz, die den PSTI-Sequenzen vorausgehen, aufweisen.

## TABELLE 1

### PSTI - Varianten

| Position |    | 17  | 18  | 19  | 20  | 21  | 29  |
|----------|----|-----|-----|-----|-----|-----|-----|
| PSTI     | 1  | Thr | Leu | Ile | Tyr | Asn | Asp |
| PSTI     | 2  | Thr | Leu | Ile | Tyr | Asp | Asn |
| PSTI     | 3  | Thr | Tyr | Glu | Tyr | Arg | Asp |
| PSTI     | 4  | Thr | Leu | Glu | Tyr | Arg | Asp |
| PSTI     | 5  | Thr | Val | Glu | Tyr | Arg | Asp |
| PSTI     | 6  | Thr | Leu | Glu | Tyr | Asn | Asp |
| PSTI     | 7  | Thr | Leu | Ile | Tyr | Arg | Asp |
| PSTI     | 8  | Thr | Val | Glu | Leu | Asn | Asp |
| PSTI     | 9  | Thr | Val | Glu | Leu | Arg | Asp |
| PSTI     | 10 | Pro | Lys | Ile | Tyr | Asp | Asn |
| PSTI     | 11 | Pro | Leu | Glu | Tyr | Arg | Asp |
| PSTI     | 12 | Pro | Val | Glu | Tyr | Arg | Asp |
| PSTI     | 13 | Thr | Ile | Glu | Tyr | Asn | Asp |
| PSTI     | 14 | Thr | Arg | Glu | Tyr | Asn | Asp |
| PSTI     | 15 | Thr | Phe | Glu | Tyr | Asn | Asp |
| PSTI     | 16 | Thr | Ala | Glu | Tyr | Asn | Asp |
| PSTI     | 17 | Thr | Val | Ile | Tyr | Asn | Asp |
| PSTI     | 18 | Thr | Ile | Ile | Tyr | Asn | Asp |
| PSTI     | 19 | Thr | Val | Ile | Tyr | Asp | Asn |
| PSTI     | 20 | Thr | Ile | Ile | Tyr | Asp | Asn |
| PSTI     | 21 | Thr | Ile | Glu | Tyr | Arg | Asp |
| PSTI     | 22 | Thr | Tyr | Ile | Tyr | Asn | Asp |
| PSTI     | 23 | Thr | Phe | Ile | Tyr | Asn | Asp |
| PSTI     | 24 | Thr | Lys | Glu | Tyr | Arg | Asp |

Die vorliegende Erfindung betrifft auch DNA's, die für die genannten Sequenzen codieren und die funktionellen Äquivalente derselben. "Funktionelle Äquivalente" bedeutet dabei, daß auch Derivate der vorstehenden DNA-Sequenz für die Expression des gleichen Proteins geeignet sein können. Es ist dem Fachmann auf diesem Gebiet bekannt, daß in einigen Codons eine, zwei oder drei der Basen durch eine andere ersetzt werden kann bzw. können, ohne daß dies einen Einfluß auf die in ein gegebenes Protein einzubauende Aminosäure hat. Um die Varianten des Peptids herzustellen, wird das Mastergen mit Hilfe der DNA-Technologie in der Weise modifiziert, daß das Codon für eine bestimmte Aminosäure in einer bestimmten Position durch das Codon für eine andere Aminosäure ersetzt ist. Um eine DNA zu erhalten, die für eine der Peptidvarianten gemäß der vorliegenden Erfindung codiert, können die Codons für die Aminosäuren in Positionen 17, 18, 19, 20 32 und 36 durch andere Codons ersetzt werden.

Bevorzugte Codons sind bei diesem Austausch solche Codons, die für die vorstehend aufgeführten Aminosäuren codieren. Je nach dem verwendeten Expressionssystem kann die DNA gemäß der Erfindung auch eine DNA darstellen, die für eines der vorstehenden Peptide codiert und zusätzlich am $NH_2$-Terminus eine Sequenz umfaßt, die für ein Leaderpeptid codiert.

Eine weitere Aufgabe gemäß der Erfindung stellt einen Expressionsvektor dar, der auch als Plasmid bezeichnet wird und die DNA, welche für eines der Peptide gemäß der vorliegenden Erfindung codiert, umfaßt. Das Plasmid dient zur Transformation von Wirtsorganismen. Die vorliegende Erfindung betrifft auch derartig transformierte Mikroorganismen. Dem Fachmann ist eine große Zahl verschiedener Mikroorganismen bekannt, die sich für die Transformation eignen. Die Art des Plasmids wird in der Hauptsache in Abhängigkeit von dem zu verwendenden Wirtsorganismus gewählt.

Der Wirtsorganismus, der mit dem Plasmid transformiert ist, das die DNA gemäß der Erfindung umfaßt, dient zur Herstellung des vorstehend genannten Peptides und der Varianten desselben. Die Herstellung umfaßt die folgenden Schritte:

(a) Züchten des Wirtsorganismus unter geeigneten Bedingungen;

(b) Gewinnung des Peptides aus der Kultur und

(c) Reinigung des Peptides.

Die Reinigung des Peptides kann nach bekannten Methoden der Proteinchemie erfolgen, z. B. durch Präzipitation, Chromatografie und Elektrophorese. Das vorstehend genannte Linkerpeptid kann so konstruiert sein, daß es die Reinigung des Fusionsproteins erleichtert. Wenn z. B. das Linkerpeptid in der Hauptsache basische oder saure Aminosäuren umfaßt, so kann sich bei der Reinigung des exprimierten Produktes die Ionenaustauschchromatografie als besonders vorteilhaft erwiesen.

Die vorliegende Erfindung betrifft auch pharmazeutische Zusammensetzungen und Präparationen, welche die vorstehend beschriebenen Peptide umfassen, und die Verwendung der genannten Peptide bei der Herstellung pharmazeutischer Zubereitungen. Diese pharmazeutischen Zubereitungen werden insbesondere bei den vorstehend angegebenen Indikationen angewandt.

Die pharmazeutischen Präparationen können außer nicht-toxischen, inerten, pharmazeutisch geeigneten Träger bzw. Zuschlagsstoffen eine oder mehrere Verbindungen gemäß der Erfindung enthalten oder aus einem oder mehreren Wirkstoffen gemäß der Erfindung bestehen.

Die vorliegende Erfindung umfaßt auch pharmazeutische Präparationen in Dosiseinheiten. Dies bedeutet, daß die Präparationen in Form einzelner Teile vorliegen, z. B. als Tabletten, Dragees, Kapseln, Pillen, Suppositorien und Ampullen, deren Gehalt an Wirkstoff einem Bruchteil oder einem Vielfachen einer Einzeldosis entspricht. Die Dosiseinheiten können z. B. eine, zwei, drei oder vier Einzeldosen, oder eine halbe, eine drittel oder eine viertel oder eine ganze Einzeldosis enthalten. Eine Einzeldosis enthält vorzugsweise die Menge an Wirkstoff, die bei einer Verabreichung gegeben wird und die üblicherweise einer ganzen oder einer halben Dosis, oder einer drittel Dosis oder einer viertel Dosis des täglichen Bedarfs entspricht.

Unter nicht-toxischen, inerten pharmazeutisch geeigneten Trägerstoffen versteht man feste, halbfeste oder flüssige Verdünnungsmittel, Füllmittel und Formulierungshilfen aller Art.

Tabletten, Dragees, Kapseln, Pillen, Granulate, Suppositorien, Lösungen, Suspensionen und Emulsionen, Pasten, Salben, Gele, Cremes, Lotionen, Pulver und Sprays können als bevorzugte pharmazeutische Präparationen genannt werden.

Tabletten, Dragees, Kapseln, Pillen und Granulate können den Wirkstoff bzw. die Wirkstoffe zusammen mit üblichen Trägerstoffen enthalten, wie z. B. (a) Füllstoffe und Streckmittel, z. B. Stärken, Lactose, Sucrose, Glucose, Mannit und Siliciumdioxid, (b) Bindemittel, z. B. Carboxymethylcellulose, Alginate, Gelatine und Polyvinylpyrrolidon, (c) anfeuchtende Mittel, z. B. Glycerin, (d) Sprengmittel, z. B. Agar-Agar, Calciumcarbonat und Natriumcarbonat, (e) Lösungsverzögerer, Paraffin und (f) Absorptionsbeschleuniger, z. B. quaternäre Ammoniumverbindungen, (g) Netzmittel, z. B. Cetylalkohol und Glycerin-monostearat, (h) Absorbenzien, z. B. Caolin und Bentonit, und (i) Gleitmittel, z. B. Talk, Calcium- und Magnesiumstearat und feste Polyethylenglykole oder Gemische der Substanzen, wie sie unter (a) bis (i) aufgeführt sind.

Die Tabletten, Dragees, Kapseln, Pillen und Granulate können mit üblichen Überzügen und Umhüllungen versehen sein, welche gegebenenfalls Opakisierungsmittel enthalten; sie können auch in solchen Zubereitungen vorliegen, die den Wirkstoff oder die Wirkstoffe vorzugsweise nur in einem bestimmten Teil des Verdauungstraktes, gegebenenfals in verzögerter Weise, freisetzen, wobei als Beispiele für geeignete Materialien zum Einbetten Polymersubstanzen und Wachse genannt werden.

Der Wirkstoff oder die Wirkstoffe, gegebenenfalls zusammen mit einem oder mehreren der vorstehend genannten Trägerstoffen, kann bzw. können auch in Form von Mikrokapseln vorliegen.

Suppositorien können neben dem Wirkstoff oder den Wirkstoffen die üblichen wasserlöslichen oder wasserlöslichen Trägerstoffe enthalten, z. B. Polyethylenglykole, Fette, z. B. Kakaofett, und höhere Ester, z.

EP 0 278 112 B1

B. $C_{14}$-Alkohol mit $C_{16}$-Fettsäuren, oder Gemische dieser Substanzen.

Salben, Pasten, Cremes und Gele können übliche Exzipienten neben dem Wirkstoff bzw. den Wirkstoffen enthalten, z. B. tierische und pflanzliche Fette, Wachse, Paraffine, Stärke, Tragant, Cellulosederivate, Polyethylenglykole, Silicone, Bentonite, Siliciumdioxid, Talk und Zinkoxid, oder Gemische dieser Substanzen.

Pulver und Sprays können übliche Trägerstoffe neben außer dem Wirkstoff oder den Wirkstoffen enthalten, z. B. Lactose, Talk, Siliciumdioxid, Aluminiumhydroxid, Calciumsilicat und Polyamidpulver, oder Gemische dieser Substanzen. Die Sprays können außerdem übliche Treibmitel enthalten, z. B. Chlorfluorkohlenwasserstoffe.

Lösungen und Emulsionen können übliche Trägerstoffe neben dem Wirkstoff oder den Wirkstoffen enthalten, wie Lösungsmittel, solubilisierende Agenzien und emulgierende Agenzien, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsamenöl, Erdnußöl, Maiskeimöl, Olivenöl, Rhizinusöl und Sesamöl, Glycerin, Glycerinformal, Tetrahydrofurfurylalkohol, Polyethylenglykole und Fettsäureester von Sorbitan, oder Gemische dieser Substanzen.

Für die parenterale Verabreichung können die Lösungen und Emulsionen auch in steriler, mit dem Blut isotoner Form vorliegen.

Suspensionen können übliche Trägerstoffe neben dem Wirkstoff oder den Wirkstoffen enthalten, wie flüssige Verdünner, z. B. Wasser, Ethylalkohol oder Propylenglykol, suspendierende Agenzien, z. B. ethoxylierte Isostearylalkohole, Polyoxyethylen-sorbit und Sorbitanester, mikrokristalline Cellulose, Aluminiummetahydroxid, Bentonit, Agar-Agar und Tragacanth, oder Gemische dieser Substanzen.

Die genannten Formulierungen können auch Farbstoffe, Konservierungsstoffe und Additive enthalten, die den Geruch und Geschmack verbessern, z. B. Pfefferminzöl und Eukalyptusöl, sowie Süssungsmittel, z. B. Saccharin.

Die therapeutisch wirksamen Verbindungen sollen vorzugsweise in den vorstehend genannten pharmazeutischen Präparationen in einer Konzentration von ca. 0,1 bis 99,5 Gew.-%, vorzugsweise von ca. 0,5 bis 95 Gew.-%, bezogen auf das Gesamtgewicht, vorliegen.

Die vorstehend genannten pharmazeutischen Präparationen können auch andere pharmazeutische Wirkstoffe neben den Verbindungen gemäß der Erfindung enthalten.

Die vorstehend genannten pharmazeutischen Präparationen werden in üblicher Weise nach bekannten Verfahren hergestellt, z. B. durch Vermischen des Wirkstoffes oder der Wirkstoffe mit dem oder den Trägerstoffen.

Die Wirkstoffe oder die pharmazeutischen Präparationen können lokal, oral, parenteral, intraperitonal und/oder rektal verabreicht werden, vorzugsweise oral oder parenteral, wie intravenös oder intramuskulär.

Im allgemeinen hat es sich sowohl in der Human- als auch in der Veterinärmedizin als vorteilhaft erwiesen, den Wirkstoff oder die Wirkstoffe gemäß der Erfindung in Gesamtmengen von ca. 0,5 bis ca. 500, vorzugsweise von 5 bis 100 mg/kg Körpergewicht alle 24 Stunden zu verabreichen, gegebenenfalls in Form von mehreren Einzelverabreichungen, um die gewünschten Ergebnisse zu erzielen. Eine Einzelverabreichung enthält den Wirkstoff oder die Wirkstoffe gemäß der Erfindung vorzugsweise in Mengen von ca. 1 bis 250, insbesondere von 3 bis 60 mg/kg Körpergewicht. Es kann jedoch erforderlich sein, von den genannten Dosen abzuweichen, und zwar insbesondere in Abhängigkeit von der Natur und dem Körpergewicht des zubehandelnden Individuums, der Natur und der Schwere der Erkrankung, der Art der Präparation und der Verabreichungsweise der Medizin sowie der Zeit oder der Zeitdauer, über die die Verabreichung stattfindet.

Somit kann es in manchen Fällen genügen, weniger als die vorstehend genannte Menge an Wirkstoff zu verabreichen, während in anderen Fällen die vorstehend genannte Menge an Wirkstoff überschritten werden muß. Die speziell erforderliche optimale Dosis und der Verabreichungstyp der Wirkstoffe können vom Fachmann aufgrund seines Fachwissens ohne weiteres bestimmt werden.

PSTI kann aus Pankreas isoliert werden, ist jedoch nicht in größeren Mengen erhältlich, insbesondere PSTI menschlichen Ursprungs.

Es wurde deshalb erkannt, daß die Anwendung der rekombinanten DNA-Technik sowie verwandter Technologien einen wirksamen Weg zur Beschaffung der notwendigen großen Mengen an h-PSTI hoher Qualität sowie auch von Varianten dieses Peptides bieten würde. Die Herstellung eines Fremdproteins in rekombinanten Bakterien ist im Hinblick auf die in vielen Fällen angetroffene Instabilität nicht trivial (B. E. Butterworth und B. D. Kovant; J. Virol. 14, (1984) 282-291, D. V. Goeddel et al; Proc. Natn. Acad. Sci, USA 76 (1979), 106-110, M. Inouye et al in "The future in nucleic acid research", I. Watanabe Hsg., (Tokyo, Academic Press) (1983). Die Instabilität stellt, insbesondere bei der Expression von Peptiden relativ kurzer kettenlänge, ein Problem dar.

7

Der menschliche Pankreas sezerniert PSTI, einen Proteaseinhibitor mit niedrigem Molekulargewicht (6,2 kd), der spezifisch Trypsin inhibiert, d. h. dieser Inhibitor besitzt eine hohe Selektivität für einen spezifischen Proteasetyp.

Ein Vorteil gemäß der Erfindung liegt in der Substitution von nur einer (oder einiger weniger) Aminosäuren in PSTI durch rekombinante DNA-Technologie, wodurch PSTI-Varianten erhalten werden, von denen gezeigt wurde, daß sie hochwirksame Proteaseinhibitoren mit einer hohen Spezifität für Leukocyten-Elastase darstellen. Außerdem sollte aufgrund des relativ niedrigen Molekulargewichtes der Elastase-PSTI-Derivat-Komplex auch die Nieren passieren. Aus diesem Grund ist eine besonders wirksame Eliminierung von extrazellulär freigesetzter Elastase zu erwarten. Die Tatsache, daß PSTI menschlichen Ursprungs ist, zusammen mit dessen niedrigem Molekulargewicht, ließ erwarten, daß die klinische Applikation von PSTI-Derivaten keine Komplikationen aufgrund der Erkennung dieser Substanzen als Fremdproteine durch das Immunsystem verursacht.

Ein weiterer wesentlicher Vorteil von PSTI, verglichen mit $\alpha_1$-PI und Antileukoprotease, liegt in der Unempfindlichkeit gegenüber oxidativer Inaktivierung durch Entzündungsprozesse, bei denen stark oxidative Agenzien gebildet und extrazellulär freigesetzt werden. Folglich sollten, im Vergleich mit $\alpha_1$-PI oder Antileukoprotease, geringere Dosen des PSTI-Derivates ausreichen, um eine vergleichbare Schutzwirkung zu erzielen.

## DIE GENSEQUENZEN

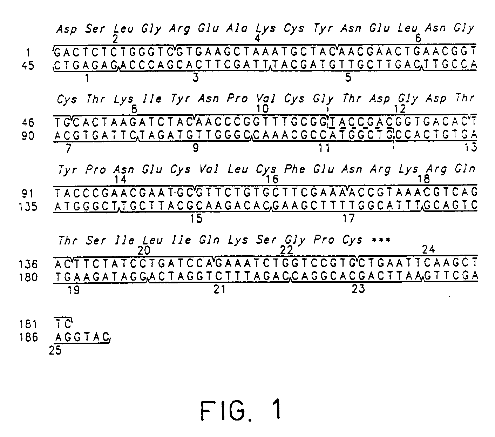

Die Aminosäuresequenz von h-PSTI wurde in die Sequenz eines DNA-Mastergens mit Hilfe solcher Codons, die bei hochexprimierten E. Coli-Genen am häufigsten angetroffen werden, zurücktranslatiert (M. Gouy und C. Gautier (1982), Nucleic Acids Res., 10, 7055-7074); siehe Fig. 1. Für die Aminosäure-Substitutionen der PSTI-Varianten (Tabelle 1) wurden die entsprechenden Codons im Mastergen ausgetauscht (siehe Tabelle 2).

Die mit einem Sternchen markierten Codons in Tabelle 2 wurden durchweg verwendet, mit folgenden Ausnahmen:

| | |
|---|---|
| Thr 26 (ACC) | in allen Genen, um eine KpnI-Stelle an den Positionen 72 bis 77 zu schaffen; |
| Lys 18 (AAG) | in PSTI-0 und -10, um eine BglII-Stelle an den Positionen 53 bis 58 zu schaffen; |
| Gly 28 (GGC) | in PSTI-2, -10, -19 und -20, um eine richtige Hybridisierung der korrespondierenden Oligonukleotid-Fragmente zu erzielen (siehe unten); |
| Pro 17 (CCT) + Leu 18 (CTA) | in PSTI-11, um eine XbaI-Stelle an den Positionen 51 bis 56 zu schaffen; |
| 5'-ende des Gens: | erstes Codon, umfaßt die zweite Hälfte der HincII-Stelle GTPyPuAC; |
| 3'-ende des Gens: | Stopp-Codon TGA, welches die EcoRI-Stelle (Position 170 bis 175) überlappt, gefolgt von einer HindIII-Stelle (Position 176 bis 181) und ein NcoI-klebriges Ende (Position 182 bis 186). |

## TABELLE 2

### Verwendete Codons

| Ala | Arg | Asp | Asn | Cys | Glu | Gln | Gly | His | Ile |
|-----|-----|-----|-----|-----|-----|-----|-----|-----|-----|
| GCA | AGA | GAC* | AAC* | UGC* | GAA* | CAA | GGA | CAC | AUA |
| GCC | AGG | GAU | AAU | UGU | GAG | CAG* | GGC | CAU | AUC* |
| GCG | CGA | | | | | | GGG | | AUU |
| GCU* | CGC | | | | | | GGU* | | |
| | CGG | | | | | | | | |
| | CGU* | | | | | | | | |

| Leu | Lys | Met | Phe | Pro | Ser | Thr | Trp | Tyr | Val | STOP |
|-----|-----|-----|-----|-----|-----|-----|-----|-----|-----|------|
| UUA | AAA* | AUG | UUC* | CCA | AGC | ACA | UGG | UAC* | GUA | UAA |
| UUG | AAG | | UUU | CCC | AGU | ACC | | UAU | GUC | UAG |
| CUA | | | | CCG* | UCA | ACG | | | GUG | UGA* |
| CUC | | | | CCU | UCC | ACU* | | | GUU* | |
| CUG* | | | | | UCG | | | | | |
| CUU | | | | | UCU* | | | | | |

Die PSTI-Gen-Konstruktion wurde wie folgt durchgeführt:

(1) Synthese von 25 kurzen Oligonukleotiden (jeweils 12 und 13 für jeden komplementären Strang) wobei für einige Varianten spezifische Fragmente synthetisiert wurden (siehe Tabelle 3). Die Reinigung des Fragmentes mit der richtigen Größe erfolgte durch Ionenaustausch-Hochleistungs-Flüssigchromatografie (HPLC).

(2) Ligieren der Fragmente unter Erhalt eines doppelsträngigen Moleküls für den Codierungsbereich von reifem PSTI (Aminosäuren 1 bis 56) mit einem "glatten" Ende am Amino-terminalen Codierungsende und einem GAT-einzelsträngigen 5′ überhängenden Ende am Carboxy-terminalen Codierungsende. Für diesen Schritt war es wichtig, die zu synthetisierenden Oligonukleotidsequenzen zu optimieren, um falsche Legierungsprodukte auf einem Minimum zu halten.

(3) Klonieren in mehreren Vektoren, z. B. pUC8, pGV451, M13mp18 zur Sequenzierung (siehe Text zu Verfahren).

(4) Schaffung weiterer Varianten über ortsspezifische Mutagenese in M13-Phagenvektoren unter Verwendung spezifischer synthetischer Oligonukleotide.

## TABELLE 3

Bedeutung der Zahlen:

| Spalte 1: | die Fragmentzahlen (siehe Fig. 1) |
|---|---|
| Spalte 2 und 3: | erste und letzte Nukleotidpositionen des Fragmentes innerhalb der Sequenz des Mastergens (5′ ⟶ 3′) |
| Spalte 4: | Nukleotidsequenz der Fragmente (5′ ⟶ 3′) |

Die Punkte zeigen Fragmente an, die von der Sequenz des Mastergens abweichen und die für die Konstruktion von Genvarianten erforderlich sind.

10

Tabelle 3 (Fortsetzung)


PSTI 1 (Segment I)


Liste der Fragmente 1 - 11

```
      1   7    1  GAGAGTC
      2   1   13  GACTCTCTGGGTC
      3  23    8  TTAGCTTCACGACCCA
      4  14   30  GTGAAGCTAAATGCTAC
      5  39   24  CAGTTCGTTGTAGCAT
      6  31   47  AACGAACTGAACGGTTG
  •   7  54   40  CAGAGTGCAACCGTT
  •   8  48   60  CACTCTGATCTAC
      9  66   55  CGGGTTGTAGAT
     10  61   74  AACCCGGTTTGCGG
     11  81   67  GTCGGTACCGCAAAC
```

Tabelle 3 (Fortsetzung)

PSTI 2 (Segment I)

Liste der Fragmente 1 - 11

```
      1    7    1   GAGAGTC
      2    1   13   GACTCTCTGGGTC
      3   23    8   TTAGCTTCACGACCCA
      4   14   30   GTGAAGCTAAATGCTAC
      5   39   24   CAGTTCGTTGTAGCAT
      6   31   47   AACGAACTGAACGGTTG
  ●   7   54   40   CAGAGTGCAACCGTT
  ●   8   48   60   CACTCTGATCTAC
  ●   9   66   55   CGGGTCGTAGAT
  ●  10   61   74   GACCCGGTTTGCGG
     11   81   67   GTCGGTACCGCAAAC
```

PSTI 3 (Segment I)

Liste der Fragmente 1 - 11

```
      1    7    1   GAGAGTC
      2    1   13   GACTCTCTGGGTC
      3   23    8   TTAGCTTCACGACCCA
      4   14   30   GTGAAGCTAAATGCTAC
      5   39   24   CAGTTCGTTGTAGCAT
      6   31   47   AACGAACTGAACGGTTG
  ●   7   54   40   GTAAGTGCAACCGTT
  ●   8   48   60   CACTTACGAATAC
  ●   9   66   55   CGGACGGTATTC
  ● 10   61   74   CGTCCGGTTTGCGG
     11   81   67   GTCGGTACCGCAAAC
```

Tabelle 3 (Fortsetzung)

PSTI 4 (Segment I)

Liste der Fragmente 1 - 11

```
     1   7   1  GAGAGTC
     2   1  13  GACTCTCTGGGTC
     3  23   8  TTAGCTTCACGACCCA
     4  14  30  GTGAAGCTAAATGCTAC
     5  39  24  CAGTTCGTTGTAGCAT
     6  31  47  AACGAACTGAACGGTTG
   • 7  54  40  CAGAGTGCAACCGTT
   • 8  48  60  CACTCTGGAATAC
   • 9  66  55  CGGACGGTATTC
   •10  61  74  CGTCCGGTTTGCGG
    11  81  67  GTCGGTACCGCAAAC
```

PSTI 5 (Segment I)

Liste der Fragmente 1 - 11

```
     1   7   1  GAGAGTC
     2   1  13  GACTCTCTGGGTC
     3  23   8  TTAGCTTCACGACCCA
     4  14  30  GTGAAGCTAAATGCTAC
     5  39  24  CAGTTCGTTGTAGCAT
     6  31  47  AACGAACTGAACGGTTG
   ‰ 7  54  40  AACAGTGCAACCGTT
   • 8  48  60  CACTGTTGAATAC
   • 9  66  55  CGGACGGTATTC
   •10  61  74  CGTCCGGTTTGCGG
    11  81  67  GTCGGTACCGCAAAC
```

Tabelle 3 (Fortsetzung)

PSTI 6 (Segment I)

Liste der Fragmente 1 - 11

```
      1    7    1   GAGAGTC
      2    1   13   GACTCTCTGGGTC
      3   23    8   TTAGCTTCACGACCCA
      4   14   30   GTGAAGCTAAATGCTAC
      5   39   24   CAGTTCGTTGTAGCAT
      6   31   47   AACGAACTGAACGGTTG
  •   7   54   40   CAGAGTGCAACCGTT
  •   8   48   60   CACTCTGGAATAC
  •   9   66   55   CGGGTTGTATTC
     10   61   74   AACCCGGTTTGCGG
     11   81   67   GTCGGTACCGCAAAC
```

PSTI 7 (Segment I)

Liste der Fragmente 1 - 11

```
      1    7    1   GAGAGTC
      2    1   13   GACTCTCTGGGTC
      3   23    8   TTAGCTTCACGACCCA
      4   14   30   GTGAAGCTAAATGCTAC
      5   39   24   CAGTTCGTTGTAGCAT
      6   31   47   AACGAACTGAACGGTTG
  •   7   54   40   CAGAGTGCAACCGTT
  •   8   48   60   CACTCTGATCTAC
  •   9   66   55   CGGACGGTAGAT
  •  10   61   74   CGTCCGGTTTGCGG
     11   81   67   GTCGGTACCGCAAAC
```

Tabelle 3 (Fortsetzung)

PSTI 13 (Segment I)

Liste der Fragmente 1 - 11

```
      1    7    1   GAGAGTC
      2    1   13   GACTCTCTGGGTC
      3   23    8   TTAGCTTCACGACCCA
      4   14   30   GTGAAGCTAAATGCTAC
      5   39   24   CAGTTCGTTGTAGCAT
      6   31   47   AACGAACTGAACGGTTG
  •   7   54   40   GATAGTGCAACCGTT
  •   8   48   60   CACTATCGAATAC
  •   9   66   55   CGGGTTGTATTC
     10   61   74   AACCCGGTTTGCGG
     11   81   67   GTCGGTACCGCAAAC
```

PSTI 14 (Segment I)

Liste der Fragmente 1 - 11

```
      1    7    1   GAGAGTC
      2    1   13   GACTCTCTGGGTC
      3   23    8   TTAGCTTCACGACCCA
      4   14   30   GTGAAGCTAAATGCTAC
      5   39   24   CAGTTCGTTGTAGCAT
      6   31   47   AACGAACTGAACGGTTG
  •   7   54   40   ACGAGTGCAACCGTT
  •   8   48   60   CACTCGTGAATAC
  •   9   66   55   CGGGTTGTATTC
     10   61   74   AACCCGGTTTGCGG
     11   81   67   GTCGGTACCGCAAAC
```

15

Tabelle 3 (Fortsetzung)

PSTI 15 (Segment I)

Liste der Fragmente 1 - 11

```
     1   7   1  GAGAGTC
     2   1  13  GACTCTCTGGGTC
     3  23   8  TTAGCTTCACGACCCA
     4  14  30  GTGAAGCTAAATGCTAC
     5  39  24  CAGTTCGTTGTAGCAT
     6  31  47  AACGAACTGAACGGTTG
   • 7  54  40  GAAAGTGCAACCGTT
   • 8  48  60  CACTTTCGAATAC
   • 9  66  55  CGGGTTGTATTC
    10  61  74  AACCCGGTTTGCGG
    11  81  67  GTCGGTACCGCAAAC
```

PSTI 16 (Segment I)

Liste der Fragmente 1 - 11

```
     1   7   1  GAGAGTC
     2   1  13  GACTCTCTGGGTC
     3  23   8  TTAGCTTCACGACCCA
     4  14  30  GTGAAGCTAAATGCTAC
     5  39  24  CAGTTCGTTGTAGCAT
     6  31  47  AACGAACTGAACGGTTG
   • 7  54  40  AGCAGTGCAACCGTT
   • 8  48  60  CACTGCTGAATAC
   • 9  66  55  CGGGTTGTATTC
    10  61  74  AACCCGGTTTGCGG
    11  81  67  GTCGGTACCGCAAAC
```

Tabelle 3 (Fortsetzung)

PSTI 17 (Segment I)

Liste der Fragmente 1 - 11

```
     1   7   1  GAGAGTC
     2   1  13  GACTCTCTGGGTC
     3  23   8  TTAGCTTCACGACCCA
     4  14  30  GTGAAGCTAAATGCTAC
     5  39  24  CAGTTCGTTGTAGCAT
     6  31  47  AACGAACTGAACGGTTG
 •   7  54  40  AACAGTGCAACCGTT
 •   8  48  60  CACTGTTATCTAC
     9  66  55  CGGGTTGTAGAT
    10  61  74  AACCCGGTTTGCGG
    11  81  67  GTCGGTACCGCAAAC
```

PSTI 18 (Segment I)

Liste der Fragmente 1 - 11

```
     1   7   1  GAGAGTC
     2   1  13  GACTCTCTGGGTC
     3  23   8  TTAGCTTCACGACCCA
     4  14  30  GTGAAGCTAAATGCTAC
     5  39  24  CAGTTCGTTGTAGCAT
     6  31  47  AACGAACTGAACGGTTG
 •   7  54  40  GATAGTGCAACCGTT
 •   8  48  60  CACTATCATCTAC
     9  66  55  CGGGTTGTAGAT
    10  61  74  AACCCGGTTTGCGG
    11  81  67  GTCGGTACCGCAAAC
```

Tabelle 3 (Fortsetzung)


PSTI 19 (Segment I)


Liste der Fragmente 1 - 11

```
   1   7   1  GAGAGTC
   2   1  13  GACTCTCTGGGTC
   3  23   8  TTAGCTTCACGACCCA
   4  14  30  GTGAAGCTAAATGCTAC
   5  39  24  CAGTTCGTTGTAGCAT
   6  31  47  AACGAACTGAACGGTTG
•  7  54  40  AACAGTGCAACCGTT
•  8  48  60  CACTGTTATCTAC
•  9  66  55  CGGGTCGTAGAT
• 10  61  74  GACCCGGTTTGCGG
  11  81  67  GTCGGTACCGCAAAC
```


PSTI 20 (Segment I)


Liste der Fragmente 1 - 11

```
   1   7   1  GAGAGTC
   2   1  13  GACTCTCTGGGTC
   3  23   8  TTAGCTTCACGACCCA
   4  14  30  GTGAAGCTAAATGCTAC
   5  39  24  CAGTTCGTTGTAGCAT
   6  31  47  AACGAACTGAACGGTTG
•  7  54  40  GATAGTGCAACCGTT
•  8  48  60  CACTATCATCTAC
•  9  66  55  CGGGTCGTAGAT
• 10  61  74  GACCCGGTTTGCGG
  11  81  67  GTCGGTACCGCAAAC
```

Tabelle 3 (Fortsetzung)

PSTI-A (Segment II)

Liste der Fragmente 12 - 25

```
   12  75  89  TACCGACGGTGACAC
 • 13  97  82  TAGCGTAAGTGTCACC
 • 14  90 105  TTACGCTAACGAATGC
   15 113  98  CACAGAACGCATTCGT
   16 106 121  GTTCTGTGCTTCGAAA
   17 129 114  TTTACGGTTTTCGAAG
   18 122 137  ACCGTAAACGTCAGAC
   19 145 130  GGATAGAAGTCTGACG
   20 138 152  TTCTATCCTGATCCA
   21 160 146  CAGATTTCTGGATCA
   22 153 167  GAAATCTGGTCCGTG
   23 174 161  AATTCAGCACGGAC
   24 168 182  CTGAATTCAAGCTTC
   25 186 175  CATGGAAGCTTG
```

PSTI-S (Segment II)

Liste der Fragmente 12 - 25

```
   12  75  89  TACCGACGGTGACAC
 • 13  97  82  TAGAGTAAGTGTCACC
 • 14  90 105  TTACTCTAACGAATGC
   15 113  98  CACAGAACGCATTCGT
   16 106 121  GTTCTGTGCTTCGAAA
   17 129 114  TTTACGGTTTTCGAAG
   18 122 137  ACCGTAAACGTCAGAC
   19 145 130  GGATAGAAGTCTGACG
   20 138 152  TTCTATCCTGATCCA
   21 160 146  CAGATTTCTGGATCA
   22 153 167  GAAATCTGGTCCGTG.
   23 174 161  AATTCAGCACGGAC
   24 168 182  CTGAATTCAAGCTTC
   25 186 175  CATGGAAGCTTG
```

Tabelle 3 (Fortsetzung)


PSTI 2, 10, 19 und 20 (Segment II)


Liste der Fragmente 12 - 25

```
● 12  75   89   TACCGACGGCAACAC
● 13  97   82   TCGGGTAAGTGTTGCC
  14  90  105   TTACCCGAACGAATGC
  15 113   98   CACAGAACGCATTCGT
  16 106  121   GTTCTGTGCTTCGAAA
  17 129  114   TTTACGGTTTTCGAAG
  18 122  137   ACCGTAAACGTCAGAC
  19 145  130   GGATAGAAGTCTGACG
  20 138  152   TTCTATCCTGATCCA
  21 160  146   CAGATTTCTGGATCA
  22 153  167   GAAATCTGGTCCCTG
  23 174  161   AATTCAGCACGGAC
  24 168  182   CTGAATTCAAGCTTC
  25 186  175   CATGGAAGCTTG
```


PSTI 21 (Segment I)


Liste der Fragmente 1 - 11

```
   1   7    1   GAGAGTC
   2   1   13   GACTCTCTGGGTC
   3  23    8   TTAGCTTCACGACCCA
   4  14   30   GTGAAGCTAAATGCTAC
   5  39   24   CAGTTCGTTGTAGCAT
   6  31   47   AACGAACTGAACGGTTG
 ● 7  54   40   GATAGTGCAACCGTT
 ● 8  48   60   CACTATCGAATAC
 ● 9  66   55   CGGACGGTATTC
 ●10  61   74   CGTCCGGTTTGCGG
  11  81   67   GTCGGTACCGCAAAC
```

Tabelle 3 (Fortsetzung)


PSTI 22 (Segment I)


Liste der Fragmente 1 - 11

```
 1   7   1  GAGAGTC
 2   1  13  GACTCTCTGGGTC
 3  23   8  TTAGCTTCACGACCCA
 4  14  30  GTGAAGCTAAATGCTAC
 5  39  24  CAGTTCGTTGTAGCAT
 6  31  47  AACGAACTGAACGGTTG
 7  54  40  GTAAGTGCAACCGTT
 8  48  60  CACTTACATCTAC
 9  66  55  CGGGTTGTAGAT
10  61  74  AACCCGGTTTGCGG
11  81  67  GTCGGTACCGCAAAC
```


PSTI 23 (Segment I)


Liste der Fragmente 1 - 11

```
 1   7   1  GAGAGTC
 2   1  13  GACTCTCTGGGTC
 3  23   8  TTAGCTTCACGACCCA
 4  14  30  GTGAAGCTAAATGCTAC
 5  39  24  CAGTTCGTTGTAGCAT
 6  31  47  AACGAACTGAACGGTTG
 7  54  40  GAAAGTGCAACCGTT
 8  48  60  CACTTTCATCTAC
 9  66  55  CGGGTTGTAGAT
10  61  74  AACCCGGTTTGCGG
11  81  67  GTCGGTACCGCAAAC
```

## Tabelle 3 (Fortsetzung)

## PSTI 24 (Segment I)

### Liste der Fragmente 1 - 11

```
 1   7   1  GAGAGTC
 2   1  13  GACTCTCTGGGTC
 3  23   8  TTAGCTTCACGACCCA
 4  14  30  GTGAAGCTAAATGCTAC
 5  39  24  CAGTTCGTTGTAGCAT
 6  31  47  AACGAACTGAACGGTTG
• 7  54  40  TTTAGTGCAACCGTT
• 8  48  60  CACTAAAGAATAC
• 9  66  55  CGGACGGTATTC
•10  61  74  CGTCCGGTTTGCGG
 11  81  67  GTCGGTACCGCAAAC
```

GENKONSTRUKTE

Die aufgezeichneten Gensequenzen wurden in Oligonukleotid-Fragmente aufgeteilt (Fig. 1). Diese Fragmente weisen eindeutige Überlappungen auf, um einen korrekten enzymatischen Zusammenbau von DNA-Doppelsträngen in einer Multi-Komponenten-Reaktion zu gewährleisten.

Die Grundstrategie zur Konstruktion von PSTI-Genvarianten sieht den separaten Zusammenbau von zwei Segmenten der Gene vor:

Segment I: aus Fragmenten 1 - 11; dies umfaßt die variablen Aminosäuren-Positionen 17 - 21

Segment II: aus Fragmenten 12 - 25; dies umfaßt die variablen Aminosäure-Positionen 29 und 32 sowie 36.

Tabelle 3 zeigt die Kombinationen von Oligonukleotid-Fragmenten, die für den Zusammenbau der verschiedenen Segmente I und II verwendet wurden. Punkte zeigen die Fragmente an, die von denen des Mastergens abweichen.

Durch enzymatische Ligierung der korrespondierenden Segmentpaare 1 und 2 erhält man die gewünschten kompletten Genvarianten. Genmultimere wurden zuerst hergestellt, indem man auch eine Ligierung glatter Enden an den 5'-Enden von Segmenten I und eine Ligierung klebriger Enden an den 3'-Enden von Segmenten II zuließ. Monomere Gene wurden dann durch Schneiden mit HincII und entweder HindIII, EcoRI oder NcoI herausgeschnitten.

METHODEN

Chemische Synthese von Oligonukleotid-Fragmenten

Alle erforderlichen Oligodesoxyribonukleotide wurden mit Hilfe der bekannten Phosphotriesterchemie auf Zellulosedisks als Segmentträger synthetisiert (Frank et al (1983), Nucleic Acids Res. 11, 4365-4377), Sie wurden mittels Ionenaustausch-HPLC auf einer Whatman-Partisil-SAX-10-Säule mit einem Triethylammoniumphosphatgradienten (pH 6,3) in 60 %igem wässrigen Formamid gereinigt.

Ligierung von Oligonukleotiden zu Segment I und II DNA-Duplices

Äquimolare Mengen (50 pmol) eines jeden Oligonukleotid-Fragmentes (siehe Tabelle 3), wurden in siliconisierten Plastikröhrchen vereinigt (Röhrchen A: Fragmente, die den oberen Strang umfassen; Röhrchen B: Fragmente, welche den unteren Strang umfassen). Fragmente 1 und 11, sowie 12 und 25, die nicht phosphoryliert werden sollten (z. B. die Enden der Duplices), wurden in einem separaten Röhrchen (Röhrchen C) vereinigt. Das Material wurde eingetrocknet. Die Oligonukleotide von Röhrchen A und B wurden bei 37°C 30 Minuten mit einem Dreifach-Überschuß an [$\gamma$-$^{32}$P]ATP (ca. 2 Ci/Millimol) über 5'-Hydroxylenden in Reaktionsgemischen von 10 $\mu$l, welche 60 mM Tris•HCl (pH 8), 6 mM MgCl$_2$, 10 mM DTE und 2 Einheiten T4 PNK (Polynukleotidkinase) enthielten, phosphoryliert. Nach quantitativer Phosphorylierung wurde T4 PNK durch Erwärmen der Röhrchen A und B für 2 Minuten auf 95°C inaktiviert und die Inhalte wurden in Röhrchen C überführt. 10 mM DTE, 0,2 mM ATP und 2 Einheiten T4 DNA-Ligase wurden zugegeben und das Gemisch 1 Stunde bei 40°C inkubiert. Nach Abkühlen auf 37°C wurden weitere 2 Einheiten T4 DNA-Ligase zugegeben und die Inkubation 1 Stunde bei 37°C, 3 Stunden bei 30°C und über Nacht bei 15°C fortgesetzt. Teile der Gemische wurden dann durch Elektrophorese auf einem 0,04 x 20 x 40 cm, 10 %igem nichtdenaturierenden Polyacrylamidgel (Acrylamid: Methylenbisacrylamid-29:1, 50 mM Tris•borat (pH 8,3) 1mM EDTA), welches auf 25°C thermostatisiert war, bei 1.000 V analysiert (BPB = Bromphenolblau wandert bis 25 cm). Proben für die Gelelektrophorese wurden in einem Gemisch aufgegeben, das 0,05 % XC, 0,05 % BPB, 10 mM EDTA und 4 M Harnstoff enthielt und wurden vor dem Auftragen nicht erwärmt. Die ursprünglichen Reaktionsgemische wurden bei -20°C gelagert bis das Ergebnis der Gelelektrophorese die Bildung eines Produktes der erwarteten Länge anzeigte.

Dann wurde die DNA-Ligase durch 15-minütiges Erwärmen auf 65°C inaktiviert. Die Ligierungsprodukte wurden aus den Reaktionsgemischen durch präparative Gelelektrophorese auf einem 1 mm dicken Gel unter den vorstehend genannten Bedingungen isoliert.

## ZUSAMMENBAU DER KOMPLETTEN PSTI-GEN-VARIANTEN

10 pmol der korrespondierenden DNA-Segmente I und II wurden vereinigt und 30 Minuten bei 37°C in einem 20 $\mu$l-Reaktionsgemisch phosphoryliert, welches 60 pmol [$\gamma$-$^{32}$P]ATP (200 Ci/mmol), 60 mM Tris•HCl (pH 8), 6 mM MgCl$_2$, 10 mM DTE und 2 Einheiten T4 PNK enthielt. Dann wurden 2 $\mu$l 1 mM ATP und 2 Einheiten T4 DNA-Ligase zugegeben, worauf eine Inkubierung bei 15°C über Nacht folgte. Das Reaktionsgemisch wurde dann auf 50 $\mu$l verdünnt, wobei die geeigneten Pufferkonditionen für den Schnitt mit Restriktionsenzymen eingestellt wurden.

Zum Beispiel wurden die PSTI-Gen-Varianten
PSTI-1-Ala-32(-1A),
PSTI-3-Pro-32-(-3P),
PSTI-6-Ser-32-(-4A) und
PSTI-5-Pro-32(5P)
gemäß dem vorstehenden Protokoll konstruiert.

## KLONIEREN VON PSTI-GEN-VARIANTEN

### Strategie

Gene wurden, wie vorstehend beschrieben, aus synthetischen Gen-Fragmenten mit verschiedenen Sequenzen in den Positionen, die für die Aminosäuren 17, 18, 19, 20, 21, 29, 32 und 36 codieren, synthetisiert. Die Nomenklatur PSTI-0 bis PSTI-24 (siehe Tabelle 1) bezieht sich auf eine spezielle Codierungssequenz für den Bereich der Aminosäuren 17 bis 29, der entweder folgt: A (für Alanin), S (für Serin), G (für Glycin) oder P (für Prolin) in Position 32.

Die Genkonstrukte sind so geplant, daß die Varianten im Segment I mit Varianten im Segment II (z. B. Varianten in Position 32) nach Spalten an einer KpnI-Stelle im Position 72 bis 76 rekombiniert werden können (Fig. 1).

Weitere Varianten wurden hergestellt entweder durch ortsspezifische Mutagenese auf den Gen-Fragmenten, welche in M13 Bakteriophagen-Vektoren kloniert wurden, und zwar unter Verwendung synthetischer Nukleotide, oder durch eine totale Gensynthese, wie dies vorstehend beschrieben wurde.

### Klonierung

Gene für die PSTI-Varianten (z. B. PSTI-1A und PSTI-3P) mit einem glatten Ende am 5'-Terminus (Amino-terminales Codierungsende) und einer HindIII-Stelle am 3'-Ende, wurden in das mit HincII, HindIII-Restriktionsenzym-behandelten Plasmid pUC8 kloniert (pUC-PSTI-1A und pUC-PSTI-3P).

PSTI-Varianten, welche außerdem am NH$_2$-Ende Methionin enthielten (z. B. Met$^{-1}$-PSTI-4A) wurden in ähnlicher Weise in pUC8-NcoI (welches den NcoI-Linker CCCATGGG, eingeführt in die HincII-Stelle, enthält) kloniert. pUC8-NcoI wurde mit NcoI-Restriktionsenzym behandelt, mit dNTP in Gegenwart von DNA-PolI (großes Fragment) aufgefüllt und im weiteren mit HindIII behandelt. Durch klonieren des Gens für die PSTI-4A-Variante in diesen Vektor wurde die NcoI-Restriktionsstelle rekonstituiert (pUC-Met-PSTI-4A).

Methoden zur Restriktionsenzymspaltung, Ligierung, Transformation und zum Screenen auf "Insertions-Inaktivierung" der β-Galactosidase-Aktivität wurden von T. Maniatis, E. G. Fritsch und J. Sambrook in Molekular cloning, Cold Spring Harbor Publications, Cold Spring Harbor, 1982, beschrieben.

Für die weitere Analyse und DNA-Sequenzierung einiger Varianten wurden die HincII-HindIII-PSTI-Fragmente in M13mp(-8, 9, 18 oder 19)-Vektoren gemäß den vom Hersteller angegebenen Instruktionen (Boehringer, Mannheim) geklont.

Die Herstellung von zusätzlichen Varianten über Rekombination

Die Restriktionsendonuclease KpnI spaltet im PSTI-Gen in allen Konstruktionen nach der Base 76 auf dem oberen Strang und nach der Base 72 auf dem unteren Strang, wodurch 4 bp-einzelsträngige 3′-überstehende Enden (GTAC) gebildet werden.

Nach der KpnI, ScaI-Doppelspaltung von pUC8-PSTI-Hybriden werden zwei Fragmente erhalten, welche in einem Fragment das 5′-Ende des Gens mit den variablen Sequenzen für die Aminosäurereste 17 bis 21, und in dem anderen Fragment das 3′-Ende des Gens mit den Variationen der Aminosäuren 29, 32 und 36 enthalten. Die erneute Ligierung von Fragmenten aus verschiedenen Plasmiden ergibt neue rekombinante pUC8-PSTI-Varianten mit neu angeordneten 5′- und 3′-Bereichen (z. B. ausgehend von PSTI-OP und PSTI-1A kann man PSTI-OA und PSTI-1P ableiten). Der Escherichia coli K-12-Stamm RR1ΔM15 und DH1 (ATCC 31343 und 33625) wurden als aufnehmende Wirte für die neu arrangierten pUC8-PSTI-Varianten verwendet.

Die Herstellung von weiteren Varianten mittels ortsspezifischer Mutagenese

Ausgangsmaterial für dieses Mutageneseverfahren sind die M13 mp9-PSTI-Klone, die hergestellt werden durch klonieren der HincII-HindIII-PSTI-Fragmente aus pUC8-PSTI-Varianten in doppelsträngigem M13 mp9-Bakteriophagen nach den Methoden von J. Messing und J. Viera (1982), Gene, 19, 269-276. Einzelsträngiger Hybrid-Bakteriophage wurde isoliert und an einen linearen negativen Strang des Bakteriophagen M13 mp9 rev. hybridisiert. Synthetischen Oligonucleotide, die die Sequenz der neuen Variante an der Stelle der Mutagenese enthalten, werden dann an diesen sogenannten "gapped duplex" hybridisiert und durch konsekutive Wirkung der Polymerase I (Klenow-Fragment, Boehringer Mannheim) und DNA-Ligase aufgefüllt und unter Bildung einer geschlossenen ringförmigen Doppelhelix, welche Basen-Fehlpaarungen an der Stelle der Mutagenese enthält, ligiert. Durch Transformieren dieser DNA in dem Mismatch-Reparatur-Mangel-E. coli-MutS-Stamm BMH 71-18 erhält man eine hohe Frequenz von M13 mp9 rev.-PSTI-Varianten, welche die neue Sequenz, bestimmt durch den synthetischen "Mutagenese-Primer", enthalten, die im folgenden in E. Coli su⁻ (amber-Suppressor-minus)-Stamm MK 30-3 selektiert werden. Das experimentelle Protokoll entspricht dem, wie es von W. Kramer et al, Nucl. Acids Res., 12 (1984), beschrieben wird. Auf diese Weise wurden PSTI-8P, -9P, -10P, -11P, -12P,-15P, -17P, -18P und -19P konstruiert.

DNA-SEQUENZIERUNG

Die DNA-Sequenzierung wurde an einzelsträngigen M13 mp8, M13 mp9, M13 mP18, M13 mp9 (rev)-PSTI-Molekülen unter Verwendung der Didesoxy-Methode, wie sie von F. Sanger et al (1977), Proc. Natn. Acad. Sci., USA 74, 5463-5467, beschrieben wird, oder für pGV451-Konstrukte mittels der spezifischen Endmarkierung und chemischen Abbaumethode nach G. Volckaert et al (1984), Gene Analysis Techniques, 1, 52-59, durchgeführt.

KLONIERUNG IN EXPRESSIONSVEKTOREN

Die Herstellung von PSTI-Peptidvarianten wurde durch Klonierung in E. coli-Sekretionsvektoren, welche Signalsequenzen von α-Amylase (B. subtilis) oder Penicillinacylase (E. coli) enthielten, durchgeführt. Die Verwendung von E. coli-Sekretionsvektoren zur Expression eines humanen sekretorischen proteins wie PSTI, hat den Vorteil, daß ein fusioniertes Signalpeptid das gewünschte Genprodukt durch die cytoplasmatische Membran in den periplasmatischen Raum führt, bei gleichzeitiger Prozessierung des Signalpeptides (G. Kreil (1981) Ann. Rev. Biochem. 50, 317-348) und Freisetzung des aktiven PSTI, entweder in der maturen Form oder als Fusionsprodukt, welches ein Linkerpeptid aus wenigen Aminosäuren am aminoterminalen Ende enthält.

Methoden für die Spaltung mit Restriktionsenzymen, das Auffüllen von 5′-überstehenden Enden mit dNTP in Gegenwart von DNA-pol I (großes Fragment), Abbau mit $S_1$-Nuclease, Gelelektrophorese von DNA, Isolierung von DNA-Fragmenten, Ligierung und Transformation, werden von T. Maniatis et al in Molecular Cloning, Cold Spring Harbor (1982) beschrieben.

Konstruktion von α-Amylase-Sekretionsvektoren pCH 233 und pCH 2331

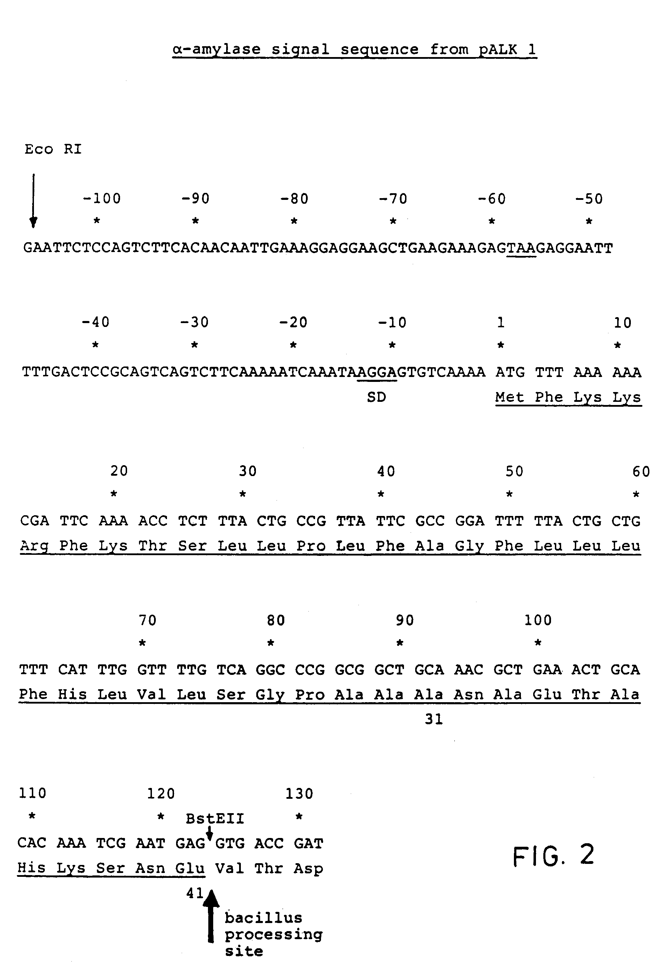

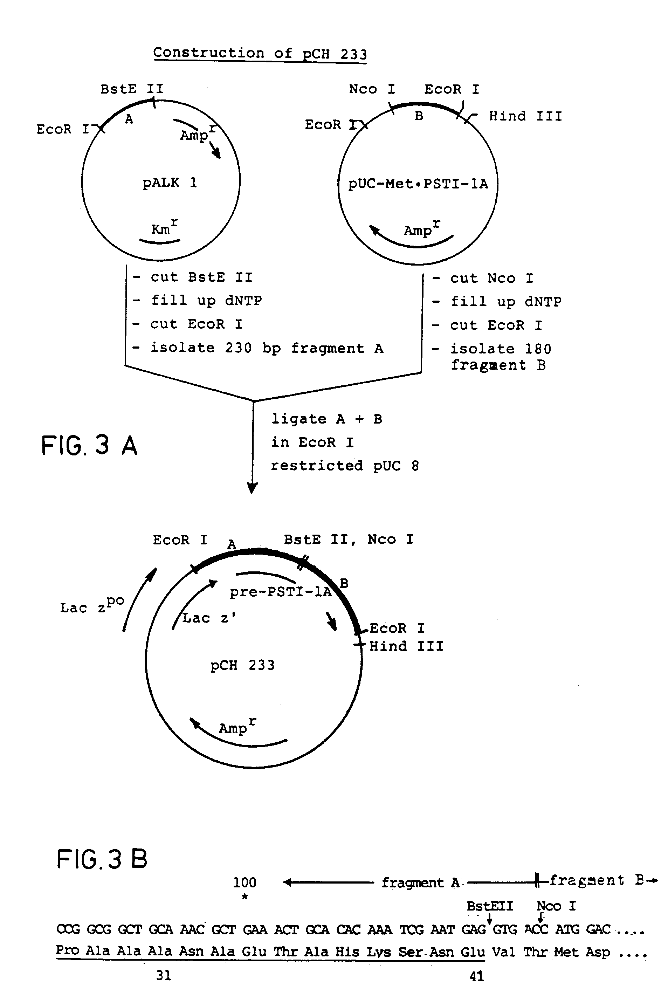

Die α-Amylase-Signalsequenz von B. subtilis wurde von Bacillus subtilis DSM 704 (Deutsche Sammlung für Mikroorganismen, Göttingen) durch Klonierung eines partiellen Sau3A-Verdaus der chromosomalen DNA in die BamH I-Stelle von pMK 3 abgeleitet (M. A. Sullivan et al, Gene (1984) 29, 21-26). Einer der Klone, welcher ein 3 Kb DNA-Fragment mit dem α-Amylase-Gen enthielt, wurde modifiziert durch Deletion von Teilen des α-Amylase-Strukturgens, um pALK1 zu ergeben (persönliche Mitteilung von Dr. M.A. Courtney; University of Rochester, N.Y., Abt. für Mikrobiologie). DNA-Sequenzen von pALK1 zeigten eine mögliche Ribosomen-Bindungsstelle (RBS) und eine Signalsequenz auf einem 230 bp EcoRI-BstEII-Fragment mit ausgeprägter Homologie zur α-Amylase aus B. subtilis 1A289 (vergleiche DNA-Sequenz in Fig. 2 mit M. Yang et al, Nucleic Acid Research (1983), 237-249). Da erwartet wurde, daß die Prozessierung der α-Amylase-Signalsequenz in B. subtilis an der Aminosäure in Position 41 erfolgt (siehe Fig. 2), wurde eine Fusion der Signalsequenz mit dem PSTI-Leseraster an der BstE II-Stelle durchgeführt. Aus diesem Grunde isolierten wir aus pALK1 (zur Verfügung gestellt von Dr. M.A. Courtney, Rochester) ein 230 bp-Fragment, welches die mögliche Shine-Dalgano-Stelle und die Signalsequenz von α-Amylase enthielt (Fragment A in Fig. 3A). Fragment B, welches das Leseraster entweder für Met-PSTI-1A oder Met-PST-4A enthielt, wurde aus pUC-Met-PSTI-1A oder pUC-Met-PSTI-4A isoliert (siehe "Klonieren von PSTI-Variationsgenen"). Die Ligierung des glatten 3′-Endes von Fragment A an das glatte 5′-Ende von B fusioniert das Leseraster der α-Amylase-Signalsequenz an das von Met-PSTI-1A oder (Met-PSTI-4A), wodurch die BstE II- und Nco I-Restriktionsstellen rekonstituiert werden (Fig. 3B). Die korrekte Orientierung des A-B-Fragmentes, relativ zu dem lac z-Promotor (s. Fig. 3A) bringt das Gen für den PSTI-Precursor unter die Kontrolle von lac $Z^{po}$ hinter das Leseraster von lac z′, welches an dem TAA-Stopp-Codon terminieren sollte (Pos. -58/-56 in der DNA-Sequenz von Fragment A, Fig. 2). Die Reinitiierung der Proteinsynthese auf der gleichen mRNA sollte nach Bindung des Ribosoms an die mögliche Shine-Dalgano-Stelle der α-Amylase ca. 50 Basen abwärts vom Stopp-Codon in Pos. -10 stattfinden (siehe Fig. 2).

Die PSTI-Produkte aus E. coli RR1ΔM15, welcher mit pCH 233 (PSTI-1A) oder pCH 2331 (PSTI-4A) transformiert ist, zeigten nach Proteinreinigung und Aminosäuresequenzierung eine Fusion von 13 Aminosäuren an das Aminoende des PSTI-Moleküls (siehe Beispiele 1 und 3). Von diesen zusätzlichen Aminosäuren, die mit asn-ala-glu-thr... beginnen, wurde festgestellt, daß sie identisch mit denen in der α-Amylase-Signalsequenz sind, die ausgehen von der Aminosäure 32 in Fig. 3B, wodurch gezeigt wird, daß die Prozessierung des PSTI-Precursors durch die E. coli-Signalpeptidase nach der Aminosäuresequenz ...pro-ala$_{29}$-ala-ala$_{31}$↓ in der Signalsequenz der Bacillus-α-amylase stattfindet.

Konstruktion von Penicillin-acylase-Sekretionsvektoren pCH 2361, pCH 2362 und pCH 2363

Eine weitere Signalsequenz für die Herstellung von PSTI-Varianten wurde von der E. coli-Penicillin-acylase (PENAC) abgeleitet, einem periplasmatischen Enzym aus E. coli ATCC 11105.

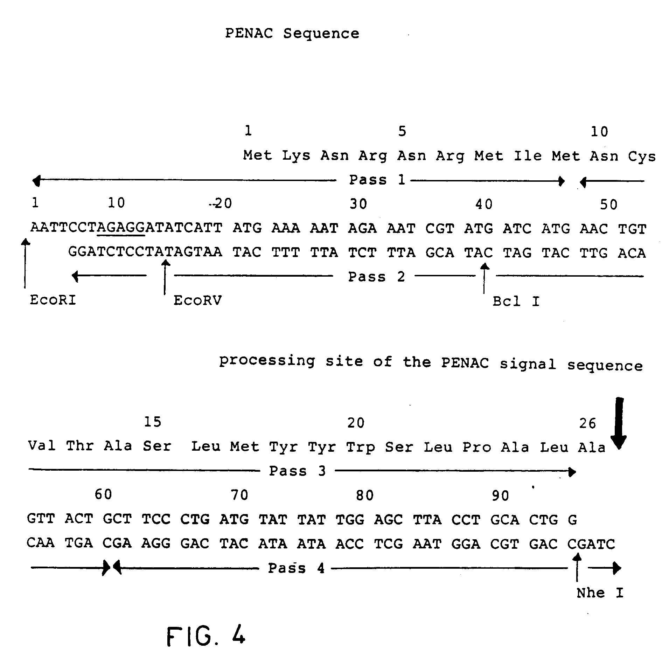

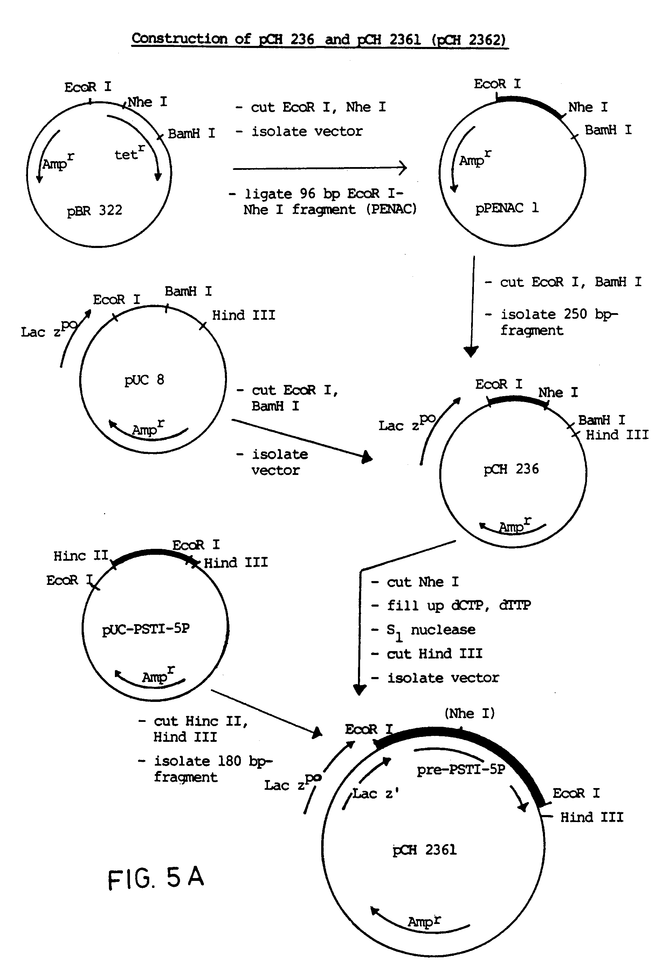

Entsprechend der DNA-Sequenz des penicillin-acylase-Gens (W. Bruns et al (1985), J. of Mol. and Appl. Genetics 3, 36-44) wurde ein DNA-Fragment synthetisiert, welches die Shine-Dalgano-Stelle und eine Signalsequenz von PENAC enthielt, welche durch Einführung einer EcoR I-Stelle aufwärts und eine EcoR V-Stelle abwärts der Shine-Dalgano-Stelle, sowie einer Bcl I-Stelle in Position 40 der Sequenz in Fig. 4 modifiziert wurde, wodurch die Aminosäure 9 der ursprünglichen PENAC-Signalsequenz von Valin zu Methionin geändert wurde. Eine NheI-Stelle wurde an der Prozessierungsstelle der PENAC-Signalsequenz bei Aminosäure 26 eingeführt. Das PENAC-DNA-Fragment wurde aus 4 DNA-Fragmenten (Pass 1-4, Fig. 4) zusammengestellt, wie dies für die PSTI-Gene beschrieben wurde; dieses Fragment wurde in einem pBR 322, der mit EcoRI und NheI geschnitten wurde, kloniert (pPENAC 1, Fig. 5A). Die Oligonucleotid-Synthese der DNA-Fragmente Pass 1-4 erfolgte mit einem Applied Biosystems Model 380 DNA-Synthesizer, entsprechend den Instruktionen des Herstellers. Die PENAC-Signalsequenz wurde dann unter die Kontrolle von lac $z^{po}$ durch Klonieren des EcoRI-BamHI-Fragments aus pPENAC1 in pUC8 gebracht (pCH 236 in Fig. 5A).

Für die Konstruktion des PENAC-Expressionsvektors mit dem Gen für PSTI-5P (pCH 2361) und für PSTI-3P (pCH 2362) wurde pCh 236 an der NheI-Stelle modifiziert (siehe Fig. 5A) und im weiteren mit HindIII nachgeschnitten. Die Ligierung des Gens für PSTI-5P (oder für PSTI-3P) in den vorbereiteten pCH 236 fusioniert die PENAC-Signalsequenz mit dem PSTI-Leseraster, wodurch sich der Expressionsvektor pCH 2361 (oder pCH 2362) ergibt. Auch hier sollte, wie vorstehend für pCH 233 beschrieben, das lac z'-Leseraster am TAG-Codon vor der Shine-Dalgano-Stelle von PENAC terminieren (Pos. 7-9 in Fig. 4).

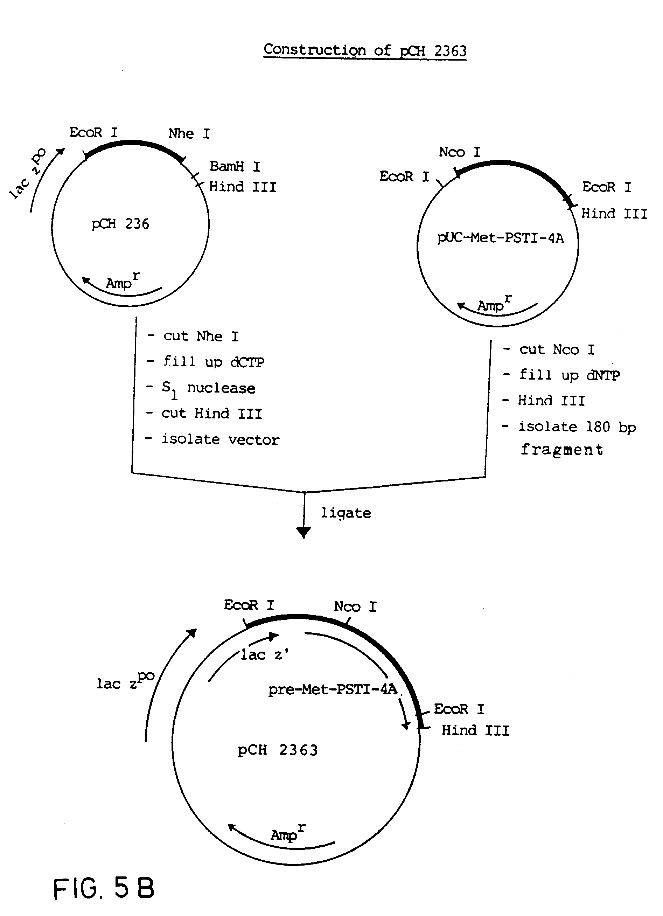

Für die Herstellung von Met-PSTI-4A (Beispiel 4) wurde pCH 2363 konstruiert, indem pCH 236 ähnlich wie vorstehend beschrieben vorbereitet wurde. Das Gen für Met-PSTI-4A wurde aus pUC-Met-PSTI-4A isoliert (Fig. 5B).

## Transformation von pCH 233, 2331, 2361, 2362 und 2363 in E. coli RR1ΔM15 oder DH₁

Bei der Transformation von kompetentem E. coli DH 1 oder RR1ΔM15 mit pCH 233, 2331, 2361-2363, dem Ausstreichen auf selektiven Medien (Agar-Platten mit 50 $\mu$g/ml Ampicillin), Minipräparationen und Restriktionsanalysen von Plasmid-DNA aus einzelnen Kolonien wurden Standardverfahren angewendet (T. Maniatis et al, 1982, Molecular Cloning - A Laboratory Manual; Cold Spring Harbor, N.Y.). Nach dem Screenen auf Restriktionsstellen, die in dem Gensegment oder in dem Expressionsvektor vorliegen, wurde die DNA-Sequenz des insertierten DNA-Fragmentes, wie vorstehend für die synthetischen Gene beschrieben, bestimmt.

## Fermentation und Induktion von E. coli RR1ΔM15, transformiert mit pCH 233, 2331, 2361, 2362 und 2363

RR1ΔM15, transformiert mit pCH 233, 2331, 2361, 2362 und 2363, wurde in Übernacht-Kulturen gezüchtet. Das Medium enthielt 3 % Rinderextrakt (Gibco), 1,5 % Hefeextrakt (Gibco) und 0,5 % $K_2HPO_4$ in destilliertem Wasser (pH 7,0) und $\mu$g/ml Ampicillin. Es wurde darauf geachtet, eine gute Belüftung zu erzielen, indem die Inkubation der Kulturen in 1.000 ml-Erlenmeyer-Kolben, die mit 100 ml Medium gefüllt waren, erfolgte.

Die Kolben wurden auf einer Kühner RC-6-U-Schüttelmaschine bei 28 und 37°C und 190 Upm geschüttelt.

Zur Herstellung der PSTI-Varianten wurden die Übernacht-Kulturen 1:100 in frischem Medium bei 37°C verdünnt und ca. 3 Stunden bis zu optischen Dichten von 1,5 A550 nm gezüchtet. Die Induktion des lac-Promotors erfolgte durch Zugabe von 1mM Isopropylthiogalactosid (Sigma) zu den Kulturen. Die Fermentation wurde für 20 Stunden bis zu 4 bis 6 A550 durchgeführt und die Zellen durch Zentrifugieren bei 8.000 Upm geerntet (10', 4°C in einer Kontron Centrikon H-401 mit Rotor A 6.14).

## ISOLIERUNG UND CHARAKTERISIERUNG DER INHIBITOREN

### Enzyme

human-Leukocyten-Elastase (HLE) und Schweine-Pankreas-Elastase (PPE) wurden von Elastin Products Company, Inc., P.O. Box 147, Pacific, Miss. 63069/USA, erhalten.

Rinder-$\alpha$-Chymotrypsin (CHT) wurde von E. Merck, Darmstadt, bezogen.

### Substrate

Die Substrate: MeOSuc-Ala-Ala-Pro-Val-pNA (HLE), Ac-Ala-Ala-Pro-Ala-pNA und Suc-Ala-Ala-Ala-pNA (PPE) und Suc-Ala-Ala-Pro-Phe-pNA (CHT) wurden von Bachem, Bubendorf/Schweiz, bezogen.

### Materialien zur Chromatografie

Sephadex[R] G-25; Sephadex[R] G-50; Sepharose[R] 4B-Cl, SP-Sephadex C-25, wurden von Deutsche Pharmacia, Freiburg, bezogen. Das hydrophobe Harz LGP 4429 ist ein Produkt der Bayer AG, Leverkusen.

### Methoden

Chymotrypsin wurde auf Sepharose[R] 4B-Cl durch Reduktion der mit Chymotrypsin gebildeten Schiff'schen Base mit Na[CNBH₃], nach Oxidation des Trägers mit Natriumperjodat immobilisiert, (G. B. Royer et al (1975), Biochem. Biophys. Res. Commun. 164, 478-484).

HPLC wurde mit Hilfe geeigneter Säulen durchgeführt. Diese sind im Text und/oder den Legenden zu den entsprechenden Abbildungen beschrieben.

BESTIMMUNG DER AMINOSÄURESEQUENZ

Ca. 0,5 bis 2 nMol des proteins wurden in 30 µl TFA aufgelöst. Die Probe wurde auf ein Glasfaserfilter aufgegeben, das mit 3 mg Polybren vorbehandelt war. Die Sequenzanalyse wurde mit Hilfe des Gasphasen-Proteinsequenzers von Applied Biosystems (Inc. USA) gemäß Hewick (R. M. Hewick, M. W. Hunkapillar, L. E. Hood, W. Dreger, 1981, J. Biol. Chem. 256, 7990-7997) durchgeführt. Die Aminosäure-Phenylthiohydantoinderivate, die in jedem Schritt freigesetzt wurden, wurden unter Verwendung einer Cyano-HPLC-Säule (DuPont) und einem Trennsystem, wie es von Beyreuther (K. Beyreuther, B. Biesler, J. Bowens, R. Dildrop, K. Neufer, K. Stüber, S. Zais, R. Ehring, P. Zabel, 1983, Modern Methods in Protein Chemistry, S. 303-325, Walter de Gruyter & Co., Berlin) beschrieben wird, analysiert. Es wurde ein Waters HPLC-System, einschließlich einer M 510-Pumpe, einem WISP 710B-Autoinjektor, einem LC-Spektrophotometer M 481 und eines SHIMADZU-Integrators CR-3A verwendet.

SÄUREHYDROLYSE UND AMINOSÄUREANALYSE

Ca. 1 nMol des Proteins gibt man in ein Pyrex-Reagenzglas, zu welchem 200 µl 6M HCl zugegeben werden, welche 0,05 % 2-Mercaptoethanol enthält (I. T. Potts Jr., 1969, Anal. Biochem. 131, 1-15). Die Reagenzgläser wurden im Vakuum abgeschmolzen und 42 Stunden bei 110°C inkubiert. Die Hydrolysate wurden schnell getrocknet, in 150 µl 0,2 M Natriumcitratpuffer, pH 2,2, wieder aufgelöst und filtriert. Die Aminosäureanalyse wurde mit einem Biotronic LC 5.000- Aminosäureanalysator durchgeführt, der mit einem Fluoreszenz-Detektor und einem SHIMADZU CR-2A-Integrator ausgestattet war. Die Aminosäuren wurden nach Reaktion mit o-Phthaldialdehyd quantifiziert, wie es im wesentlichen von Benson beschrieben wird (J. R. Benson, P. E. Hare, 1975, Proc. Natl. Acad. Sci., USA, 72, 619-622).

Enzymtests und Assays auf die Inhibitionswirkung in Kulturfiltraten

Kulturfiltrate wurden wie folgt behandelt:
10 µl Tween 80-Lösung (0,5 %) und 50 µl Perchlorsäure (70 %) wurden zu 1 ml Kulturfiltrat zugegeben. Nach 20 Minuten wurden diese Proben zentrifugiert und die klaren Überstände durch Zugabe von 225 µl gesättigte Lösung an Tris-Base neutralisiert. Aliquote Teile dieser Lösungen wurden entnommen, um die Inhibitionswirkungen zu bestimmen.

Die Bedingungen der Enzymassays sind in Tabelle 4 wiedergegeben. Im allgemeinen wurden die Proben mit einem geeigneten Puffervolumen verdünnt und darauf das Enzym zugegeben. Nach der Präinkubationszeit wurde Substrat zugegeben (die Substrate wurden in DMSO in einer Konzentration von 0,1 M aufgelöst und mit Puffer verdünnt, um die Konzentration der Stammlösung zu ergeben). Die Freisetzung von p-Nitroanilin aus den Substraten wurde bei 405 nm bestimmt und zwar entweder kontinuierlich oder nach Stoppen der Reaktion durch Zugabe von Essigsäure. 100 %-Werte bildeten solche Proben, bei denen keine Inhibitoren zugegeben wurden. Die prozentuale Inhibierung wurde entsprechend der folgende Formel berechnet:

$$\% \text{ Inhibierung} = 100 \times \left(1 - \frac{\text{OD in Gegenwart des Inhibitors}}{\text{OD in Abwesenheit des Inhibitors}}\right)$$

Die absoluten Mengen der Inhibitoren konnten mit Hilfe von Kalibrierungskurven erst dann berechnet werden, wenn geringe Mengen der gereinigten Substanzen zugänglich waren.

Die Mikroorganismen der Beispiele 3 und 6 dieser Anmeldung wurden bei der National Collection of Industrial Bacteria (NCIB), Torry Research Station, P. O. Box 31, 135 Abbey Road, Aberdeen AB9 8DG, Schottland, Grossbritannien, unter der jeweiligen Bezeichnung NCIB 12365 und NCIB 12364 hinterlegt.

Beispiel 1

Isolierung von Asn$^{-13}$-Ala$^{-12}$-Glu$^{-11}$-Thr$^{-10}$-Ala$^{-9}$-His$^{-8}$-Lys$^{-7}$-Ser$^{-6}$-Asn$^{-5}$-Glu$^{-4}$-Val$^{-3}$-Thr$^{-2}$-Met$^{-1}$-Leu$^{18}$-Glu$^{19}$-Arg$^{21}$-Ala$^{32}$-PSTI (PSTI-4A[Minifusion])

10 l der Fermentationsbrühe von RR1ΔM15/pCH 2331 wurden mit 350 ml Perchlorsäure (70 %) behandelt. Der Niederschlag wurde durch Zentrifugieren entfernt. Der Überstand wurde mit 8 N KOH-

Lösung neutralisiert. Der KClO$_4$-Niederschlag wurde durch Filtration mit Hilfe eines Büchnertrichters nach Stehenlassen bei 4°C abgesaugt.

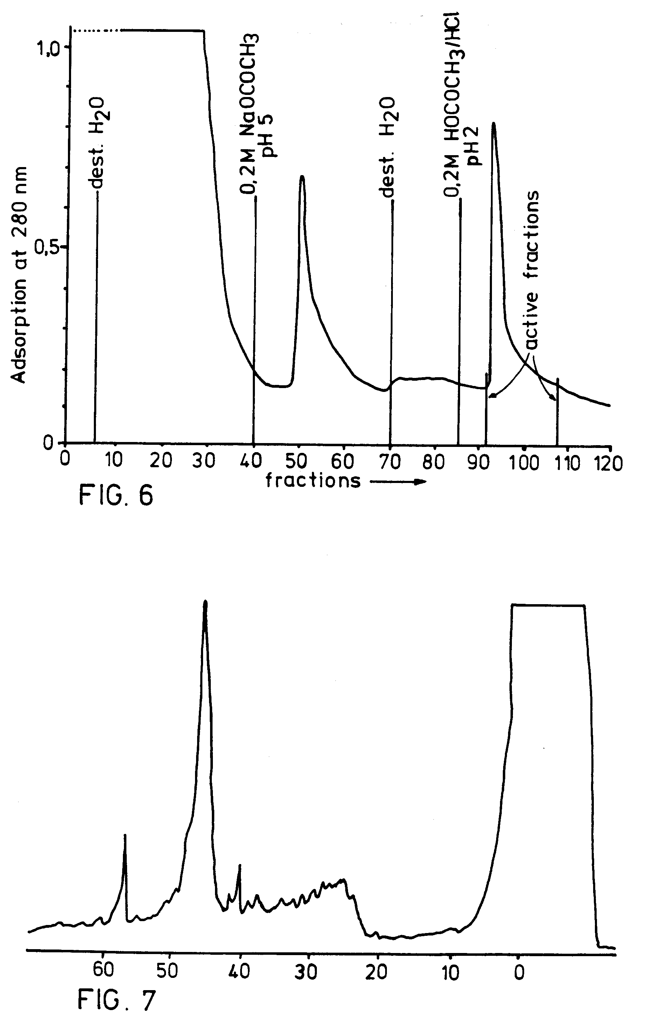

Das Filtrat wurde über ein Gelbett aus einem Chymotrypsin-Sepharose-4B-Cl-Konjugat bei 4°C (7 x 9 cm) filtriert. Das Gel wurde mit Wasser gewaschen, bis die Extinktion der Eluate bei 280 nm gegen Null ging, dann mit 500 ml 0,2 M Acetatpuffer (pH 5) und 500 ml Wasser gewaschen. Schließlich wurde das Gel mit 0,2 M Essigsäure, deren pH mit HCl auf pH 2,0 eingestellt war, eluiert. Das Elutionsprofil ist in Fig. 6 wiedergegeben. Die aktiven Fraktionen (wie im Leukocyten-Elastase-Inhibierungs-Assay gemessen) wurden vereinigt, durch Zugabe von festem Natriumacetat auf pH 4 eingestellt und durch Rotationsverdampfung konzentriert. Salze und geringfügige Verunreinigungen wurden schließlich durch präparative HPLC über einer RP-318 Säule entfernt. Das Elutionsprofil ist in Fig. 7 wiedergegeben. Die Reinheit einer jeden Fraktion wurde durch analytische HPLC bestimmt.

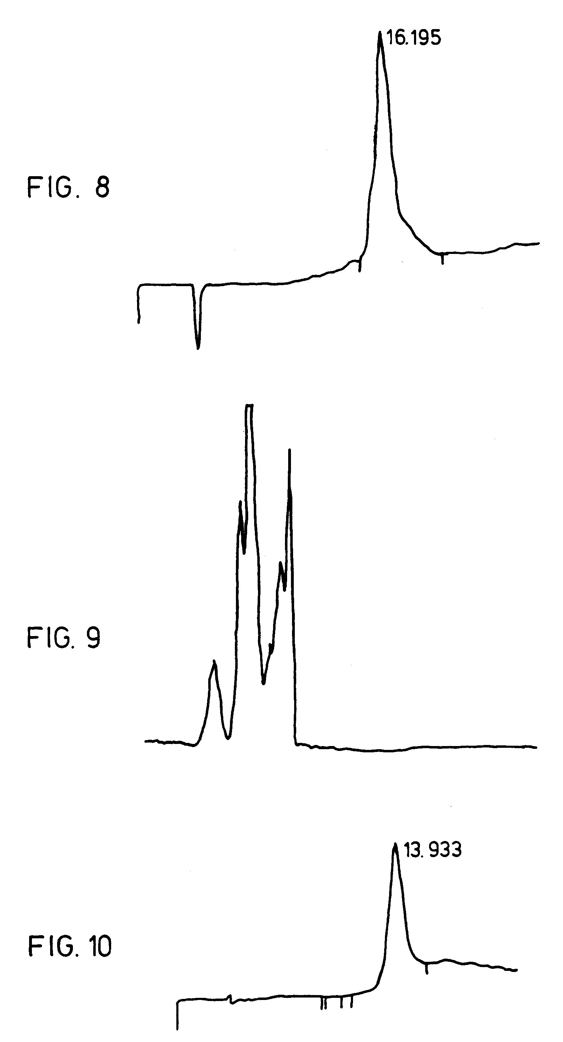

Gemäß diesen Ergebnissen wurden drei Pools bereitet, nämlich die Fraktionen 42-44, 45-46 und 47-49, welche nach Lyophilisierung 6 mg, 12 mg und 6 mg lieferten. Die höchste Reinheit wurde in der mittleren Fraktion erreicht; ein analytisches HPLC-Diagramm davon ist in Fig. 8 wiedergegeben.

Die Daten der Aminosäure-Zusammensetzung, der Sequenzanalyse und das Inhibitionsspektrum sind in den Tabellen 5 bis 7 aufgeführt.

Beispiel 2

Herstellung von Leu$^{18}$-Glu$^{19}$-Arg$^{21}$-Ala$^{32}$-PSTI (PSTI-4A) aus dem Minifusionsprotein durch BrCN-Spaltung

2,93 mg (0,46 μMol) des PSTI-4A-Minifusionsproduktes (Beispiel 1) wurden in 1 ml 70 %iger Ameisensäure aufgelöst. Die Spaltung erfolgte durch Zugabe von 3 mg Bromcyan und Inkubation über 18 Stunden bei Raumtemperatur unter Stickstoffatmosphäre im Dunkeln. Die Reaktion wurde durch Verdünnung mit 10 ml Wasser gestoppt. Das Wasser und die flüchtigen Nebenprodukte wurden durch Gefriertrocknung entfernt. Leu$^{18}$-Glu$^{19}$-Arg$^{21}$-Ala$^{32}$-PSTI (PSTI-4A) wurde von den Nebenprodukten und dem ungespaltenen Fusionsprotein durch Gelchromatografie auf einer Sephadex$^R$ G50 (Superfine)-Säule (2,5 x 90 cm) in 1 % Ameisensäure abgetrennt. Die Fraktionen wurden vereinigt und zwar entsprechend ihren Retentionszeiten auf einer hipore Biorad RP-318 (4,6 x 250 mm)-Säule, Lösung A: 0,1 % TFA; Lösung B: 0,1 % TFA/60 % Acetonitril, Fließgeschwindigkeit 1 ml/Min., Raumtemperatur, Nachweis bei 210 nm; Gradient: 0 bis 60 % B.

Die Fraktionen wurden lyophilisiert und durch erneute Chromatografie auf der gleichen Säule unter den gleichen Bedingungen gereinigt. Die aktiven Fraktionen wurden gesammelt und lyophilisiert. Die Reinheit wurde mittels eines analytischen HPLC-Laufes von Leu$^{18}$-Glu$^{19}$-Arg$^{21}$-Ala$^{32}$-PSTI (PSTI-4A), wie dies in Beispiel 1 beschrieben ist, überprüft. Der Inhibitor wurde charakterisiert durch Aminosäureanalyse, N-terminale Sequenzierung, HPLC und Elastase-Inhibitionsassay (Tabellen 5 bis 7).

Beispiel 3

Isolierung von Asn$^{-13}$-Als$^{-12}$-Glu$^{-11}$-Thr$^{-10}$-Ala$^{-9}$-His$^{-8}$-Lys $^{-7}$-Ser$^{-6}$-Asn$^{-5}$-Glu$^{-4}$-Val$^{-3}$-Thr$^{-2}$-Met$^{-1}$, Leu$^{18}$-Ala$^{32}$-PSTI (PSTI-1A[Minifusion])

4 l der Fermentationsbrühe von RR1ΔM15/pCH 233 wurden mit 120 ml Perchlorsäure (70 %) behandelt und der gebildete Niederschlag durch Zentrifugieren (30 Min., 4°C, 4.000 Upm, Beckman J-6) entfernt. Der Überstand wurde mit 8 N NaOH-Lösung auf pH 8,6 eingestellt und über ein Chymotrypsin-Sepharose-4B-Cl-Konjugat (8 x 10 cm) filtriert. Das Gel wurde mit 0,2 M Tris-HCl-Puffer (pH 8,6) gewaschen, bis die Extinktion des Durchlaufes bei 280 nm die Basislinie erreichte. Daraufhin wurde das Gel nacheinander mit Wasser und 0,1 M Essigsäure, eingestellt auf pH 2 mit HCl, eluiert.

Die Fraktionen, die die inhibitorische Aktivität (gemessen gegen Leukocyten-Elastase) aufwiesen, wurden vereinigt, mit 8 N NaOH-Lösung auf pH 2,7 eingestellt und über SP-Sephadex C-25 (2,5 x 35 cm), welches vorher mit 0,15 % Trifluoressigsäure (eingestellt auf pH 2,7 mit (NaOH-Lösung) equilibriert worden war, aufgegeben.

Nach Waschen mit dem Ausgangspuffer wurde die Säule mit einem linearen Gradienten von NaCl von 0 bis 1 M im Ausgangspuffer eluiert. Die Fraktionen wurden entsprechend ihrer inhibitorischen Aktivität und den Ergebnissen der analytischen HPLC-Läufe (RP-318, Biorad) vereinigt. Der Pool wurde durch Ultrafiltration über eine UM2-Membran (Amicon) konzentriert und die endgültige Reinigung mit Hilfe präparativer HPLC-Chromatografie über RP-318 (Gradient 0 bis 60 % Acetonitril in 0,15 % Trifluoressigsäure über 60 Min.) erreicht. Es wurden zwei Fraktionen geschnitten und lyophilisiert, wobei 8,7 mg einer homogenen Substanz und 1,9 mg mit kleineren Verunreinigungen erhalten wurden. Die analytischen Daten (Aminosäureanalyse, Sequenzanalyse und Inhibitionswirkung) sind in den Tabellen 5 bis 7 wiedergegeben.

Eine Bromcyan-Spaltung des PSTI-1A-Minifusionsproteins wurde, wie in Beispiel 2 beschrieben, durchgeführt und ergab Leu$^{18}$-Ala$^{32}$-PSTI (PSTI-1A).

Beispiel 4

Isolierung von Met$^{-1}$-Leu$^{18}$-Glu$^{19}$-Arg$^{21}$-Ala$^{32}$-PSTI (Met-PSTI-4A)

1 l Fermentationsbrühe von RR1ΔM15/pCH 2363 wurde mit Perchlorsäure behandelt, zentrifugiert und neutralisiert, wie dies in Beispiel 1 beschrieben ist, und schließlich über ein Chymotrypsin-Sepharose-4B/Cl-Konjugat (2,5 x 10 cm) gegeben. Das Gel wurde mit Wasser, Acetatpuffer und Wasser, wie dies in Beispiel 1 beschrieben ist, gewaschen. Die Desorption des Inhinbitors erfolgte durch Eluierung mit 0,2 M Essigsäure, pH 2 (eingestellt mit HCl). Die Fraktionen, welche den Inhibitor enthielten, wurden vereinigt, durch Zugabe von NaOH-Lösung auf pH 4 eingestellt und im Rotationsverdampfer auf 5 ml konzentriert. Die endgültige Reinigung wurde durch präparative HPLC über RP-318 unter Verwendung eines Gradienten von 0 bis 60 % Acetonitril in 0,15 % Trifluoressigsäure erzielt. Die Lyophilisierung der vereinigten aktiven Fraktionen ergab 1,8 mg Substanz. Weitere Daten (Aminosäure-Zusammensetzung, Sequenzanalyse und Inhibitionswirdung) sind in Tabellen 5 bis 7 wiedergegeben.

Beispiel 5

Isolierung von Tyr$^{18}$-Glu$^{19}$-Arg$^{21}$-PSTI (PSTI-3P)

750 ml Fermentationsbrühe von RR1ΔM15/pCH 2362 wurden mit 25 ml Perchlorsäure behandelt und nach 30 Minuten zentrifugiert. Der Überstand wurde durch Zugabe von 4 N KOH-Lösung neutralisiert; KC10$_4$ wurde nach 6 Stunden durch Filtration entfernt. Das Filtrat wurde auf eine Chymotrypsin-Sepharose-4B-Cl-Säule (2,5 x 7 cm) gegeben, die im folgenden mit Wasser und 0,2 M Essigsäure gewaschen wurde. Der Inhibitor wurde mit Hilfe von 0,2 M Essigsäure, eingestellt auf pH 1,8 mit HCl, desorbiert. Die aktiven Fraktionen wurden vereinigt, mit NaOH-Lösung auf pH 3,5 eingestellt und durch Rotationsverdampfung konzentriert. Ein geringfügiger Niederschlag wurde durch Zentrifugieren entfernt und der Überstand über RP-318 in einer präparativen HPLC gegeben. Durch Gefriertrocknung der aktiven Fraktionen wurden ca. 0,8 mg des Inhibitors erhalten, der sich bei der analytischen HPLC als homogen erwies. Analytische Daten (Aminosäure-Analyse, Sequenzanalyse und Inhibitionswirkung) sind in den Tabellen 5 bis 7 aufgeführt.

Beispiel 6

Isolierung von Val$^{18}$-Glu-$^{19}$-Arg$^{21}$-PSTI (PSTI-5P)

Zu 3 l der Fermentationsbrühe von RR1ΔM15/pCH 2361 wurden 100 ml Perchlorsäure (70 %) gegeben. Nach 30 Minuten wurde der Niederschlag abfiltriert und der Überstand mit 8 N NaOH-Lösung auf pH 7 gebracht. Das Filtrat wurde dann über ein hydrophobes Harz (Lewapol LGP 4429, Bayer AG), welches vorher mit 0,1 N HCl, Methanol und Wasser gewaschen worden war (∅: 3 cm, Betthöhe: 40 cm) gegeben. Das Harz wurde mit Wasser gewaschen und anschließend mit einem linearen Gradienten von 0 bis 70 % Methanol eluiert. Die Inhibitionswirkung wurde in jeder Fraktion bestimmt (Leukocyten-Elastase-Inhibitionsassay nach Entfernung von Methanol durch Verdampfung). Die aktiven Fraktionen wurden vereinigt und durch Verdampfung im Vakuum konzentriert. Das Konzentrat wurde durch Zugabe von Trifluoressigsäure auf pH 2,7 angesäuert und auf SP-Sephadex-25 (2,5 x 10 cm), welches vorher mit 0,15 % Trifluoressigsäure (pH 2,7 eingestellt mit NaOH) equilibriert worden war, gegeben.

Die Säule wurde dann mit einem linearen Gradienten von 0 bis 1 M NaCl im Ausgangspuffer entwickelt. Die Fraktionen mit Inhibitionswirkung wurden vereinigt, neutralisiert, konzentriert und durch Filtration über Sephadex G-25 mit 0,01 M Phosphatpuffer, pH 7,0, entsalzt. Die aktiven Fraktionen wurden auf pH 2,7 eingestellt und erneut auf SP-Sephadex C-25 mit einem linearen Gradienten von 0,1 bis 0,5 M NaCl bei pH 2,7 chromatografiert.

Die aktiven Eluate wurden auf pH 4,0 eingestellt, konzentriert und weiter gereinigt, indem sie über RP-318 in einer präparativen HPLC chromatografiert wurden. Dieser Schritt wurde einmal wiederholt. Eine endgültige Reinigung wurde erzielt durch präparative HPLC auf einer Mono-S-Säule von Pharmacia und einer nachfolgenden erneuten Chromatografie über RP-318 (Fig. 9). Analytische HPLC-Diagramme des gereinigten Produktes sind in Fig. 10 wiedergegeben. Weitere analytische Daten (Aminosäure-Zusammensetzung, Sequenz, Inhibitionswirkung) sind in Tabellen 5 bis 7 aufgeführt.

**TABELLE 4**

| | Leukocyten-Elastase (human) | Pankreas Elastase (Schwein) | α-Chymotrypsin (Rind) |
|---|---|---|---|
| Puffer | 0,2 M Tris, pH 8 + 0,1 % Tween 80 | 0,2 M Tris, pH 8 + 0,1 % Tween 80 + 0,05 % Na-Azid | 0,2 M Tris, pH 8 + 0,05 % Tween 80 |
| Gesamtvolumen (nach Zugabe von Substrat) | 0,65 ml | 0,65 ml | 0,60 ml |
| Enzymmenge pro Assay | 50 ng | 25 - 50 ng | 50 ng |
| Präinkubationszeit Temperatur | 30 Min. Raumtemperatur | 30 - 150 Min. Raumtemperatur | 30 Min. Raumtemperatur |
| Substrate: Formel Stammlösung Menge pro Assay Zitat | MeO-Suc-Ala-Ala-Pro-Val-pNA 0,065 M 0,1 ml 1) | Ac-Ala-Ala-Pro-Ala-pNA 0,065 M 0,1 ml 2) | Suc-Ala-Ala-Pro-Phe-pNA 0,005 M 0,04 ml 3) |
| Inkubationstemperatur | 30°C | 30°C | Raumtemperatur |

1) Nakjima, K., Powers, J.C., Castillo, M.J., Ashe, B.M., Zimmerman, M., J. Biol. Chem. 254 (1979) 4027
2) Zimmerman, M., Ashe, B.M., Biochim. Biophys. Acta 480 (1977), 241
3) DelMar, E.G., Largman, C., Brodrick, J.W., Geokas, M.C., Anal. Biochem. 99 (1979) 316

**TABELLE 5: Aminosäureanalyse verschiedener Inhibitoren**

| Amino säure | PSTI-4A | PSTI-4A [mini-fusion] | PSTI-1A | PSTI-1A [minifusion] | Met-PSTI-4A | PSTI-5P | PSTI-3P |
|---|---|---|---|---|---|---|---|
| Asp | 6.45 (7) | 8.46 (9) | 8.09 (8) | 9.95 (10) | 6.91 (7) | 7.23 (7) | 6.70 (7) |
| Thr | 3.66 (4) | 5.48 (6) | 4.00 (4) | 5.50 (6) | 3.71 (4) | 3.67 (4) | 3.61 (4) |
| Ser | 2.83 (3) | 3.70 (4) | 2.79 (3) | 3.82 (4) | 3.24 (3) | 3.38 (3) | 2.47 (3) |
| Glu | 6.83 (7) | 9.45 (9) | 6.65 (6) | 8.61 (8) | 7.09 (7) | 7.79 (7) | 7.14 (7) |
| Gly | 4.91 (5) | 5.50 (5) | 5.37 (5) | 5.75 (5) | 5.52 (5) | 5.55 (5) | 4.94 (5) |
| Ala | 2.08 (2) | 3.70 (4) | 2.20 (2) | 4.0 (4) | 2.21 (2) | 1.19 (1) | 1.06 (1) |
| Val | 2.08 (2) | 3.02 (3) | 2.00 (2) | 3.19 (3) | 2.00 (2) | 2.83 (3) | 1.92 (2) |
| Met | - (0) | 0.88 (1) | - | 0.85 (1) | 1.14 (1) | - | - (0) |
| Ile | 1.80 (2) | 1.78 (2) | 2.73 (3) | 3.01 (3) | 1.86 (2) | 1.91 (2) | 1.70 (2) |
| Leu | 4.60 (5) | 3.13 (5) | 4.62 (5) | 5.03 (5) | 5.26 (5) | 4.00 (4) | 3.47 (4) |
| Tyr | 2.66 (3) | 3.13 (3) | 2.95 (3) | 2.65 (3) | 2.50 (3) | 3.01 (3) | 3.50 (4) |
| Phe | 1.00 (1) | 1.14 (1) | 1.22 (1) | 1.25 (1) | 0.76 (1) | 1.35 (1) | 1.00 (1) |
| His | - (0) | 0.88 (1) | - | 1.15 (1) | - | - | - (0) |
| Lys | 2.91 (3) | 4.25 (4) | 2.84 (3) | 3.65 (4) | 3.42 (3) | 3.21 (3) | 2.67 (3) |
| Arg | 3.60 (4) | 4.11 (4) | 3.20 (3) | 3.22 (3) | 3.80 (4) | 4.22 (4) | 3.60 (4) |

Die Aminosäuren wurden durch Derivatisierung mit o-Phthalaldehyd nach der Trennung gemessen; Cys und Pro wurden nicht bestimmt.

EP 0 278 112 B1

TABELLE 6: N-terminale Aminosäuresequenzanalyse der PSTI-Varianten, wie sie in Tabelle 5 beschrieben sind

| Derivate | Position | | | | | | | | | | | | | | | | | | | | | |
|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 |
| PSTI-4A | Asp | Ser | Leu | Gly | Arg | Glu | Ala | Lys | Cys | Tyr | Asn | Glu | Leu | Asn | Gly | Cys | Thr | Leu | Glu | Tyr | Arg | Pro |
| PSTI-4A minifusion | Asn | Ala | Glu | Thr | Ala | His | Lys | Ser | Asn | Glu | Val | Thr | Met | Asp | Ser | Leu | Gly | Arg | Glu | Ala | Lys | Cys |
| PSTI-1A | Asp | Ser | Leu | Gly | Arg | Glu | Ala | Lys | Cys | Tyr | Asn | Glu | Leu | Asn | Gly | Cys | Thr | Leu | Ile | Thr | Asn | Pro |
| PSTI-1A minifusion | Asn | Ala | Glu | Thr | Ala | His | Lys | Ser | Asn | Glu | Val | Thr | Met | Asp | Ser | Leu | Gly | Arg | Glu | Ala | Lys | Cys |
| Met$^{-1}$-PSTI-4A | Met | Asp | Ser | Leu | Gly | Arg | Glu | Ala | Lys | Cys | Tyr | Asn | Glu | Leu | Asn | Gly | Cys | Thr | Leu | Glu | Tyr | Arg |
| PSTI-5P | Asp | Ser | Leu | Gly | Arg | Glu | Ala | Lys | Cys | Tyr | Asn | Glu | Leu | Asn | Gly | Cys | Thr | Val | Glu | Tyr | Arg | Pro |
| PSTI-3P | Asp | Ser | Leu | Gly | Arg | Glu | Ala | Lys | Cys | Tyr | Asn | Glu | Leu | Asn | Gly | Cys | Thr | Tyr | Glu | Tyr | Arg | Pro |

TABELLE 7: Inhibitionswirkung von PSTI-Varianten gemäß der Erfindung. Es werden die Inhibitormengen, die 50 % Inhibition ergeben, angegeben.

| Enzyme<br>Inhibitor | α-Chymotrypsin<br>(Rind)<br>(50 ng/Assay) | Granulocyten-Elastase<br>(Human)<br>(120 ng/Assay) | Pankreas-Elastase<br>(Schwein)<br>(500 ng/Assay) |
|---|---|---|---|
| PSTI-4A [Minifusion]<br>gemäß Beispiel 1 | 6 ng | 15 ng | 68 ng |
| PSTI-4A<br>gemäß Beispiel 2 | 7 ng | 17 ng | 75 ng |
| PSTI-1A<br>gemäß Beispiel 3 | 100 ng | 18 ng | 1200 ng |
| Met-PSTI-4A<br>gemäß Beispiel 4 | 8 ng | 16 ng | 73 ng |
| PSTI-3P<br>gemäß Beispiel 5 | 7 ng | > 5000 ng | - |
| PSTI-5P<br>gemäß Beispiel 6 | 100 ng | 15 ng | 70 ng |

LEGENDEN ZU DEN ABBILDUNGEN

Fig. 1: Nucleotid- und entsprechende Aminosäuresequenz des PSTI-Mastergens. Die Klammern und Zahlen verweisen auf die synthetischen Oligonucleotide, die beim Zusammensetzen des Gens verwendet wurden. Die gestichelte Linie zeigt die Teilung des Gens in Segment I (erster Teil) und Segment II (zweiter Teil) an.

Fig. 2: DNA-Sequenz des 230 bp EcoRI-BstEII-Fragmentes von pALK1, welches die mögliche Shine-Dalgano-Stelle (SD) um die Position -10 und die Signalsequenz von α-Amylase enthält, ausgehend von Position 1. Die mögliche Prozessierungsstelle für das Exoenzym ist an der Aminosäure 41 angegeben.

Fig. 3A: Fragment A wurde aus pALK1 isoliert durch Schneiden des Plasmids mit BstEII und aufgefüllt mit dNTP in Gegenwart von DNApolI (großes Fragment). Die DNA wurde mit Phenol behandelt, mit Ethanol präzipitiert und anschließend mit EcoRI behandelt. Das 230 bp DNA-Fragment (A) wurde nach elektrophoretischer Auftrennung auf einem 2 %-Agarosegel isoliert. Das 180 bp-Fragment B wurde in ähnlicher Weise aus pUC-Met-PSTI-1A (oder aus pUC-Met-PSTI-4A zur Konstruktion von pCH 2331) isoliert durch Restriktion mit NcoI, aufgefüllt mit dNTP, mit Ethanol präzipitiert und schließlich mit EcoRI behandelt. Beide Fragmente A und B wurden in einen mit EcoRI geschnittenen pUC8 ligiert.

Fig. 3B: zeigt die Sequenz an der Verbindungsstelle zwischen der α-Amylase-Signalsequenz (Fragment A) und dem Met-PSTI-Gen (Fragment B), entweder für Met-PSTI-1A oder -4A. Die Prozessierung der Signalsequenz in E. coli findet zwischen der Aminosäure 31 und 32 statt (siehe Beispiel 1).

Fig. 4: Fig. 4 zeigt das synthetische EcoRI-NheI-DNA-Fragment (PENAC), welches die mögliche Shine-Dalgano-Stelle (um Position 10) und die modifizierte Signalsequenz von E. coli Penicillinacylase mit der Prozessierungsstelle an der Aminosäure $Ala_{26}$ enthält.

Fig. 5A: Die synthetische PENAC-Sequenz (96 bp EcoRI-NheI-Fragment, vgl. Fig. 4) wurde in pBR322 kloniert, der mit EcoRI und NheI geschnitten wurde, um pPENAC1 zu ergeben. pPENAC1 wurde mit EcoRI und BamHI behandelt; das 250 bp DNA-Fragment, welches eine Shine-Dalgano-Stelle und die PENAC-Signalsequenz enthält, wurde in pUC8, welches mit EcoRI und BamHI geschnitten worden war, ligiert, um pCH 236 zu ergeben.

Zur Konstruktion von pCH 2361 (oder pCH 2362) wurde pCH 236 mit NheI geschnitten und mit dCTP und dTTP in Gegenwart von DNApolI (großes Fragment) aufgefüllt. Die DNA wurde mit Phenol behandelt und mit Ethanol präzipitiert; eine S1-Nuclease-Reaktion wurde durchgeführt, um ein Codon mit glattem Ende für $Ala_{26}$ an der Spaltstelle der Signalpeptidase in der PENAC-Signalsequenz (vgl. Fig. 4) zu bilden. Nach DNA-Präzipitation wurde pCH 236 im weiteren mit HindIII behandelt und der Vektor wurde nach Elektrophorese auf einem 0,8 %igen Agarosegel isoliert.

Das Gen für PSTI-5P wurde durch Restriktion von pUC-PSTI-5P mit HincII und HindIII erzeugt; das 180 bp-Fragment wurde nach Elektrophorese auf einem 2 %igen Agarosegel isoliert. Das Gen für PSTI-3P wurde in ähnlicher Weise aus pUC-PSTI-3P isoliert. Die Ligierung des 180 bp HincII-HindIII-Fragmentes aus pUC-PSTI-5P (oder pUC-PSTI-3 ) in pCH 236 ergab pCH 2361 (oder pCH 2362).

Fig. 5B: pCH 236 wurde mit NheI geschnitten und mit dCTP in Gegenwart von DNApolI (großes Fragment) aufgefüllt. Nach DNA-Präzipitation wurde eine S1-Nuclease-Behandlung durchgeführt, um ein glattes Ende zu bilden. Der Vektor wurde im weiteren mit HindIII behandelt und nach Elektrophorese auf einem 0,8 %igen Agarosegel isoliert. Das Gen für Met-PSTI-4A wurde aus pUC-Met-PSTI-4A durch Restriktion mit NcoI erzeugt und mit dNTP aufgefüllt. Nach Äthanolfällung wurde die DNA im weiteren mit HindIII behandelt und das 180 bp-Fragment nach Elektrophorese aus einem 2 %igen Agarosegel isoliert.

Fig. 6: Isolierung von PSTI-4A [Minifusion] durch Affinitätschromatografie unter Verwendung einer Säule (7 x 9 cm) mit immobilisiertem Chymotrypsin bei 4°C. Es wurden Fraktionen von 11 ml gesammelt. Auf der Abszisse ist die Nummer der Reagensgläser wiedergegeben. Die Ordinate gibt die Extinktion bei 280 nm wieder. Die Säule wurde mit Wasser (bis Fraktion 40) und dann mit 0,2 M Acetatpuffer, pH 5,0 (bis Fraktion 70) und destilliertem Wasser (bis Fraktion 85) entwickelt. Die endgültige Desorption wurde mit 0,2 M Essigsäure/Salzsäure, pH 2,0, erzielt. Der Inhibitor wurde in den Fraktionen 92 bis 108 eluiert.

Fig. 7: Präparative HPLC von PSTI-4A [Minifusion] mit Eluaten der Chymotrypsin-Affinitätssäule (Fraktionen 92 bis 108) auf einer RP-318-Säule (250 x 4,6 mm) (Biorad). Die Eluierung erfolgte mit einem linearen Gradienten von 0,15 % Trifluoressigsäure und 60 % Acetonitril, welches 0,15 % Trifluroessigsäure enthielt (2 Stunden). Fließgeschwindigkeit: 1 ml/Min. bei 150 bar; Detektion bei 220 nm. Auf der Ordinate ist die Extinktion bei 220 nm wiedergegeben; die Abszisse gibt die Fraktionsnummer an. Der Inhibitor wurde in den Fraktionen 44 bis 50 eluiert.

Fig. 8: Analytische HPLC von PSTI-4A [Minifusion] auf einer Ultra-pak TSK SP-5 PW-Säule mit Vorsäule (75 x 7,5 mm LKB).

BEDINGUNGEN

| | |
|---|---|
| Lösungsmittel A: | 0,15 % Trifluoressigsäure und |
| | 0,1 M $Na_2SO_4$, |
| | 10 % Methanol, pH 2,7; |
| Lösungsmittel B: | 0,15 % Trifluoressigsäure und |
| | 0,6 M $Na_2SO_4$, |
| | 10 % Methanol, pH 2,7; |
| Gradient: | 0 - 5 Min. 5 % B |
| | 5 - 30 Min. 15 - 100 % B |
| | 30 - 33 Min. 100 - 15 % B |
| | 33 - 40 Min. 15 % B |
| Fließgeschwindigkeit: | 1,5 ml/Min. bei 20 bar |
| Detektion: | 215 nm |

Fig. 9: Endreinigung von PSTI-5P durch präparative HPLC auf RP-318 (250 x 4,6 mm) (Biorad). Eluierung mit einem linearen Gradienten von 0,15 % Trifluoressigsäure und 0,15 % Trifluoressigsäure, 60 % Acetonitril in 2 Stunden. Fließgeschwindigkeit: 1 ml/Min. bei 150 bar; Detektion bei 280 nm. Der Inhibitor wurde in den Fraktionen 20 bis 27 eluiert.

Fig. 10: Analytische HPLC von PSTI-5P unter Verwendung einer Bio-Sil$^R$ TSK CM-3-SW-Säule (75 × 7,5 mm) (Biorad).

BEDINGUNGEN

| | |
|---|---|
| Lösungsmittel A: | 0,15 % Trifluoressigsäure und |
| | 0,1 M $Na_2SO_4$, |
| | 10 % Methanol |
| Lösungsmittel B: | 0,15 % Trifluoressigsäure und |
| | 0,6 M $Na_2SO_4$, |
| | 10 % Methanol |
| Gradient: | 0 - 5 Min. 15 % B |
| | 5 - 30 Min. 15 - 100 % B |
| | 30 - 32 Min. 100 - 15 % B |
| | 32 - 40 Min. 15 % B |
| Fließgeschwindigkeit: | 1,5 ml/Min. bei 22 bar |
| Detektion: | 215 nm |

**Patentansprüche**

1. Peptid, welches im wesentlichen die Sequenz des humanen pankreatischen, sekretorischen Trypsininhibitors aufweist, dadurch gekennzeichnet, daß eine oder mehrere der Aminosäuren in den Positionen 17, 18, 19, 20, 32 und 36 durch eine beliebige, natürlich vorkommende Aminosäure ersetzt sind.

2. Peptid gemäß Anspruch 1, dadurch gekennzeichnet, daß es die Aminosäuren Glu, Asp oder Leu in Position 13; Glu, Asp oder Asn in Position 14; Thr, Pro, Ser, Arg, Leu oder Met in Position 17; Leu, Met, Val, Gln, Ser, Ala, Thr, Ile, Tyr, Phe, Arg, Trp oder Lys in Position 18; Glu, Asp, Leu oder Ile in Position 19; Tyr, Phe, Asp, Asn, Leu, Glu, Gln, His oder Arg in Position 20; Pro, Gly, Ser, Asn, Ala, Asp, Val, Arg oder His in Position 32; Asn, Asp, Ala, Ser, Gly, Tyr, Val oder Glu in Position 36 umfaßt.

3. Peptid nach einem oder mehreren der Ansprüche 1 bis 2, ausgewählt aus der Gruppe

| | | | | | | | |
|---|---|---|---|---|---|---|---|
| PSTI | 1 | Thr-17 | Leu-18 | Ile-19 | Tyr-20 | Asn-21 | Asp-29 |
| PSTI | 2 | Thr-17 | Leu-18 | Ile-19 | Tyr-20 | Asp-21 | Asn-29 |
| PSTI | 3 | Thr-17 | Tyr-18 | Glu-19 | Tyr-20 | Arg-21 | Asp-29 |
| PSTI | 4 | Thr-17 | Leu-18 | Glu-19 | Tyr-20 | Arg-21 | Asp-29 |
| PSTI | 5 | Thr-17 | Val-18 | Glu-19 | Tyr-20 | Arg-21 | Asp-29 |
| PSTI | 6 | Thr-17 | Leu-18 | Glu-19 | Tyr-20 | Asn-21 | Asp-29 |
| PSTI | 7 | Thr-17 | Leu-18 | Ile-19 | Tyr-20 | Arg-21 | Asp-29 |
| PSTI | 8 | Thr-17 | Val-18 | Glu-19 | Leu-20 | Asn-21 | Asp-29 |
| PSTI | 9 | Thr-17 | Val-18 | Glu-19 | Leu-20 | Arg-21 | Asp-29 |
| PSTI | 10 | Pro-17 | Lys-18 | Ile-19 | Tyr-20 | Asp-21 | Asn-29 |
| PSTI | 11 | Pro-17 | Leu-18 | Glu-19 | Tyr-20 | Arg-21 | Asp-29 |
| PSTI | 12 | Pro-17 | Val-18 | Glu-19 | Tyr-20 | Arg-21 | Asp-29 |
| PSTI | 13 | Thr-17 | Ile-18 | Glu-19 | Tyr-20 | Asn-21 | Asp-29 |
| PSTI | 14 | Thr-17 | Arg-18 | Glu-19 | Tyr-20 | Asn-21 | Asp-29 |
| PSTI | 15 | Thr-17 | Phe-18 | Glu-19 | Tyr-20 | Asn-21 | Asp-29 |
| PSTI | 16 | Thr-17 | Ala-18 | Glu-19 | Tyr-20 | Asn-21 | Asp-29 |
| PSTI | 17 | Thr-17 | Val-18 | Ile-19 | Tyr-20 | Asn-21 | Asp-29 |
| PSTI | 18 | Thr-17 | Ile-18 | Ile-19 | Tyr-20 | Asn-21 | Asp-29 |
| PSTI | 19 | Thr-17 | Val-18 | Ile-19 | Tyr-20 | Asp-21 | Asn-29 |
| PSTI | 20 | Thr-17 | Ile-18 | Ile-19 | Tyr-20 | Asp-21 | Asn-29 |
| PSTI | 21 | Thr-17 | Ile-18 | Glu-19 | Tyr-20 | Arg-21 | Asp-29 |
| PSTI | 22 | Thr-17 | Tyr-18 | Ile-19 | Tyr-20 | Asn-21 | Asp-29 |
| PSTI | 23 | Thr-17 | Phe-18 | Ile-19 | Tyr-20 | Asn-21 | Asp-29 |
| PSTI | 24 | Thr-17 | Lys-18 | Glu-19 | Tyr-20 | Arg-21 | Asp-29 |

wobei es im weiteren in Position 13 Glu, Asp oder Leu, in Position 14 Glu, Asp oder Asn, in Position 32 Pro, Gly, Ser, Asn, Ala, Asp, Val, Arg oder His und in Position 36 Asn, Asp, Ala, Ser, Gly, Tyr, Val oder Glu, umfasst.

4. Peptid nach einem oder mehreren der Ansprüche 1 bis 3, welches eine Aminosäure oder eine Peptidsequenz aufweist, die dem Asp in Position 1 vorangeht.

5. Peptid nach einem oder mehreren der Ansprüche 1 bis 4, dadurch gekennzeichnet, daß es durch rekombinante DNA-Technologie hergestellt wurde.

6. DNA, welche für das Peptid nach einem oder mehreren der Ansprüche 1 bis 4 codiert.

7. Expressionsvektor, dadurch gekennzeichnet, daß dieser die DNA nach Anspruch 6 umfaßt.

8. Wirtsorganismus, welcher mit dem Expressionsvektor nach Anspruch 7 transformiert ist.

9. Verwendung des Wirtsorganismus nach Anspruch 8 zur Herstellung des Peptides nach einem oder mehreren der Ansprüche 1 bis 4.

10. Verfahren zur Herstellung des Peptides nach einem oder mehreren der Ansprüche 1 bis 4, welches die folgenden Schritte umfaßt:
a) Züchten eines Wirtsorganismus unter geeigneten Bedingungen,
b) Gewinnen des Peptides aus der Kultur,
c) Reinigen des Peptides,
dadurch gekennzeichnet, daß der wirtsorganismus mit dem Expressionsvektor nach Anspruch 7 transformiert wird.

11. Verwendung des Peptides nach einem oder mehreren der Ansprüche 1 bis 4 zur Herstellung von Arzneimitteln.

12. Pharmazeutische Zubereitungen, dadurch gekennzeichnet, daß sie das Peptid nach einem oder mehreren der Ansprüche 1 bis 4 umfassen.

# EP 0 278 112 B1

**Claims**

1. Peptide which essentially has the sequence of human pancreatic secretory trypsin inhibitor, characterised in that one or more of the amino acids in the positions 17, 18, 19, 20, 32 and 36 are replaced by any desired naturally occurring amino acid.

2. Peptide according to Claim 1, characterised in that it includes the amino acids Glu, Asp or Leu in position 13; Glu, Asp or Asn in position 14; Thr, Pro, Ser, Arg, Leu or Met in position 17; Leu, Met, Val, Gln, Ser, Ala, Thr, Ile, Tyr, Phe, Arg, Trp or Lys in position 18; Glu, Asp, Leu or Ile in position 19; Tyr, Phe, Asp, Asn, Leu, Glu, Gln, His or Arg in position 20; Pro, Gly, Ser, Asn, Ala, Asp, Val, Arg or His in position 32; Asn, Asp, Ala, Ser, Gly, Tyr, Val or Glu in position 36.

3. Peptide according to one or more of Claims 1 and 2, selected from the group consisting of

| PSTI | 1 | Thr-17 | Leu-18 | Ile-19 | Tyr-20 | Asn-21 | Asp-29 |
|------|-----|--------|--------|--------|--------|--------|--------|
| PSTI | 2 | Thr-17 | Leu-18 | Ile-19 | Tyr-20 | Asp-21 | Asn-29 |
| PSTI | 3 | Thr-17 | Tyr-18 | Glu-19 | Tyr-20 | Arg-21 | Asp-29 |
| PSTI | 4 | Thr-17 | Leu-18 | Glu-19 | Tyr-20 | Arg-21 | Asp-29 |
| PSTI | 5 | Thr-17 | Val-18 | Glu-19 | Tyr-20 | Arg-21 | Asp-29 |
| PSTI | 6 | Thr-17 | Leu-18 | Glu-19 | Tyr-20 | Asn-21 | Asp-29 |
| PSTI | 7 | Thr-17 | Leu-18 | Ile-19 | Tyr-20 | Arg-21 | Asp-29 |
| PSTI | 8 | Thr-17 | Val-18 | Glu-19 | Leu-20 | Asn-21 | Asp-29 |
| PSTI | 9 | Thr-17 | Val-18 | Glu-19 | Leu-20 | Arg-21 | Asp-29 |
| PSTI | 10 | Pro-17 | Lys-18 | Ile-19 | Tyr-20 | Asp-21 | Asn-29 |
| PSTI | 11 | Pro-17 | Leu-18 | Glu-19 | Tyr-20 | Arg-21 | Asp-29 |
| PSTI | 12 | Pro-17 | Val-18 | Glu-19 | Tyr-20 | Arg-21 | Asp-29 |
| PSTI | 13 | Thr-17 | Ile-18 | Glu-19 | Tyr-20 | Asn-21 | Asp-29 |
| PSTI | 14 | Thr-17 | Arg-18 | Glu-19 | Tyr-20 | Asn-21 | Asp-29 |
| PSTI | 15 | Thr-17 | Phe-18 | Glu-19 | Tyr-20 | Asn-21 | Asp-29 |
| PSTI | 16 | Thr-17 | Ala-18 | Glu-19 | Tyr-20 | Asn-21 | Asp-29 |
| PSTI | 17 | Thr-17 | Val-18 | Ile-19 | Tyr-20 | Asn-21 | Asp-29 |
| PSTI | 18 | Thr-17 | Ile-18 | Ile-19 | Tyr-20 | Asn-21 | Asp-29 |
| PSTI | 19 | Thr-17 | Val-18 | Ile-19 | Tyr-20 | Asp-21 | Asn-29 |
| PSTI | 20 | Thr-17 | Ile-18 | Ile-19 | Tyr-20 | Asp-21 | Asn-29 |
| PSTI | 21 | Thr-17 | Ile-18 | Glu-19 | Tyr-20 | Arg-21 | Asp-29 |
| PSTI | 22 | Thr-17 | Tyr-18 | Ile-19 | Tyr-20 | Asn-21 | Asp-29 |
| PSTI | 23 | Thr-17 | Phe-18 | Ile-19 | Tyr-20 | Asn-21 | Asp-29 |
| PSTI | 24 | Thr-17 | Lys-18 | Glu-19 | Tyr-20 | Arg-21 | Asp-29 |

it additionally including in position 13 Glu, Asp or Leu, in position 14 Glu, Asp or Asn, in position 32 Pro, Gly, Ser, Asn, Ala, Asp, Val, Arg or His and in position 36 Asn, Asp, Ala, Ser, Gly, Tyr, Val or Glu.

4. Peptide according to one or more of Claims 1 to 3, which contains an amino acid or a peptide sequence which precedes the Asp in position 1.

5. Peptide according to one or more of Claims 1 to 4, characterised in that it has been prepared by recombinant DNA technology.

6. DNA which codes for the peptide according to one or more of Claims 1 to 4.

7. Expression vector, characterised in that this includes the DNA according to Claim 6.

8. Host organism, which is transformed with the expression vector according to Claim 7.

9. Use of the host organism according to Claim 8 for the production of the peptide according to one or more of Claims 1 to 4.

37

**10.** Process for the production of the peptide according to one or more of Claims 1 to 4, which includes the following steps:

a) culturing of a host organism under suitable conditions,

b) recovery of the peptide from the culture,

c) purification of the peptide,

characterised in that the host organism is transformed with the expression vector according to Claim 7.

**11.** Use of the peptide according to one or more of Claims 1 to 4 for the production of medicaments.

**12.** Pharmaceutical preparations, characterised in that they include the peptide according to one or more of Claims 1 to 4.

**Revendications**

**1.** Peptide qui présente essentiellement la séquence de l'inhibiteur sécrétoire pancréatique humain de la trypsine, qui se caractérise par le fait qu'on remplace un ou plusieurs des amino-acides dans les positions 17, 18, 19, 20, 32 et 36 par n'importe quel amino-acide existant naturellement.

**2.** Peptide selon la revendication 1, caractérisé en ce qu'il englobe les amino-acides Glu, Asp ou Leu en position 13; Glu, Asp ou Asn en position 14; Thr, Pro, Ser, Arg, Leu ou Met en position 17, Leu, Met, Val, Gln, Ser, Ala, Thr, Ile, Tyr, Phe, Arg, Trp ou Lys en position 18; Glu, Asp, Leu ou Ile en position 19; Tyr, Phe, Asp, Asn, Leu, Glu, Gln, His ou Arg en position 20; Pro, Gly, Ser, Asn, Ala, Asp, Val, Arg ou His en position 32; Asn, Asp, Ala, Ser, Gly, Tyr, Val ou Glu en position 36.

**3.** Peptide selon l'une ou plusieurs des revendications 1 à 2, choisi parmi le groupe

| PSTI | 1 | Thr-17 | Leu-18 | Ile-19 | Tyr-20 | Asn-21 | Asp-29 |
|------|----|--------|--------|--------|--------|--------|--------|
| PSTI | 2 | Thr-17 | Leu-18 | Ile-19 | Tyr-20 | Asp-21 | Asn-29 |
| PSTI | 3 | Thr-17 | Tyr-18 | Glu-19 | Tyr-20 | Arg-21 | Asp-29 |
| PSTI | 4 | Thr-17 | Leu-18 | Glu-19 | Tyr-20 | Arg-21 | Asp-29 |
| PSTI | 5 | Thr-17 | Val-18 | Glu-19 | Tyr-20 | Arg-21 | Asp-29 |
| PSTI | 6 | Thr-17 | Leu-18 | Glu-19 | Tyr-20 | Asn-21 | Asp-29 |
| PSTI | 7 | Thr-17 | Leu-18 | Ile-19 | Tyr-20 | Arg-21 | Asp-29 |
| PSTI | 8 | Thr-17 | Val-18 | Glu-19 | Leu-20 | Asn-21 | Asp-29 |
| PSTI | 9 | Thr-17 | Val-18 | Glu-19 | Leu-20 | Arg-21 | Asp-29 |
| PSTI | 10 | Pro-17 | Lys-18 | Ile-19 | Tyr-20 | Asp-21 | Asn-29 |
| PSTI | 11 | Pro-17 | Leu-18 | Glu-19 | Tyr-20 | Arg-21 | Asp-29 |
| PSTI | 12 | Pro-17 | Val-18 | Glu-19 | Tyr-20 | Arg-21 | Asp-29 |
| PSTI | 13 | Thr-17 | Ile-18 | Glu-19 | Tyr-20 | Asn-21 | Asp-29 |
| PSTI | 14 | Thr-17 | Arg-18 | Glu-19 | Tyr-20 | Asn-21 | Asp-29 |
| PSTI | 15 | Thr-17 | Phe-18 | Glu-19 | Tyr-20 | Asn-21 | Asp-29 |
| PSTI | 16 | Thr-17 | Ala-18 | Glu-19 | Tyr-20 | Asn-21 | Asp-29 |
| PSTI | 17 | Thr-17 | Val-18 | Ile-19 | Tyr-20 | Asn-21 | Asp-29 |
| PSTI | 18 | Thr-17 | Ile-18 | Ile-19 | Tyr-20 | Asn-21 | Asp-29 |
| PSTI | 19 | Thr-17 | Val-18 | Ile-19 | Tyr-20 | Asp-21 | Asn-29 |
| PSTI | 20 | Thr-17 | Ile-18 | Ile-19 | Tyr-20 | Asp-21 | Asn-29 |
| PSTI | 21 | Thr-17 | Ile-18 | Glu-19 | Tyr-20 | Arg-21 | Asp-29 |
| PSTI | 22 | Thr-17 | Tyr-18 | Ile-19 | Tyr-20 | Asn-21 | Asp-29 |
| PSTI | 23 | Thr-17 | Phe-18 | Ile-19 | Tyr-20 | Asn-21 | Asp-29 |
| PSTI | 24 | Thr-17 | Lys-18 | Glu-19 | Tyr-20 | Arg-21 | Asp-29 |

dans lequel il englobe, en outre : en position 13, Glu, Asp ou Leu; en position 14, Glu, Asp ou Asn; en position 32, Pro, Gly, Ser, Asn, Ala, Asp, Val, Arg ou His; et en position 36, Asn, Asp, Ala, Ser, Gly, Tyr, Val ou Glu.

**4.** Peptide selon l'une ou plusieurs des revendications 1 à 3, qui présente un amino-acide ou une séquence peptidique qui précède l'amino-acide Asp en position 1.

**5.** Peptide selon l'une ou plusieurs des revendications 1 à 4, caractérisé en ce qu'on le prépare par la technologie de l'ADN recombinant.

**6.** ADN qui code pour le peptide selon l'une ou plusieurs des revendications 1 à 4.

**7.** Vecteur d'expression, caractérisé en ce que ce dernier englobe l'ADN selon la revendication 6.

**8.** Organisme hôte qui est transformé avec le vecteur d'expression selon la revendication 7.

**9.** Utilisation de l'organisme hôte selon la revendication 8, pour la préparation du peptide selon l'une ou plusieurs des revendications 1 à 4.

**10.** Procédé pour la préparation du peptide selon l'une ou plusieurs des revendications 1 à 4, qui englobe les étapes ci-après :
   a) cultiver un organisme hôte dans des conditions appropriées;
   b) récupérer le peptide hors de la culture;
   c) purifier le peptide,
caractérisé en ce que l'organisme hôte est transformé avec le vecteur d'expression selon la revendication 7.

**11.** Utilisation du peptide selon l'une ou plusieurs des revendications 1 à 4, pour la préparation de médicaments.

**12.** Préparations pharmaceutiques caractérisées en ce qu'elles englobent le peptide selon l'une ou plusieurs des revendications 1 à 4.

```
     Asp Ser Leu Gly Arg Glu Ala Lys Cys Tyr Asn Glu Leu Asn Gly
              2                   4       6
  1  GACTCTCTGGGTCGTGAAGCTAAATGCTACAACGAACTGAACGGT
 45  CTGAGAGACCCAGCACTTCGATTTACGATGTTGCTTGACTTGCCA
          1            3                  5

     Cys Thr Lys Ile Tyr Asn Pro Val Cys Gly Thr Asp Gly Asp Thr
              8                  10          12
 46  TGCACTAAGATCTACAACCCGGTTTGCGGTACCGACGGTGACACT
 90  ACGTGATTCTAGATGTTGGGCCAAACGCCATGGCTGCCACTGTGA
        7            9            11              13

     Tyr Pro Asn Glu Cys Val Leu Cys Phe Glu Asn Arg Lys Arg Gln
              14              16                  18
 91  TACCCGAACGAATGCGTTCTGTGCTTCGAAAACCGTAAACGTCAG
135  ATGGGCTTGCTTACGCAAGACACGAAGCTTTTGGCATTTGCAGTC
                   15                  17

     Thr Ser Ile Leu Ile Gln Lys Ser Gly Pro Cys •••
              20                  22              24
136  ACTTCTATCCTGATCCAGAAATCTGGTCCGTGCTGAATTCAAGCT
180  TGAAGATAGGACTAGGTCTTTAGACCAGGCACGACTTAAGTTCGA
        19                21                23

181  TC
186  AGGTAC
       25
```

# FIG. 1

α-amylase signal sequence from pALK 1

Eco RI

```
          -100        -90        -80        -70        -60        -50
            *          *          *          *          *          *
GAATTCTCCAGTCTTCACAACAATTGAAAGGAGGAAGCTGAAGAAAGAGTAAGAGGAATT
```

```
          -40        -30        -20        -10          1         10
            *          *          *          *          *          *
TTTGACTCCGCAGTCAGTCTTCAAAAATCAAATAAGGAGTGTCAAAA ATG TTT AAA AAA
                                       SD              Met Phe Lys Lys
```

```
           20         30         40         50         60
            *          *          *          *          *
CGA TTC AAA ACC TCT TTA CTG CCG TTA TTC GCC GGA TTT TTA CTG CTG
Arg Phe Lys Thr Ser Leu Leu Pro Leu Phe Ala Gly Phe Leu Leu Leu
```

```
           70         80         90        100
            *          *          *          *
TTT CAT TTG GTT TTG TCA GGC CCG GCG GCT GCA AAC GCT GAA ACT GCA
Phe His Leu Val Leu Ser Gly Pro Ala Ala Ala Asn Ala Glu Thr Ala
                                              31
```

```
110        120        130
  *          *  BstEII    *
CAC AAA TCG AAT GAG↓GTG ACC GAT
His Lys Ser Asn Glu Val Thr Asp
             41↑
              │ bacillus
              │ processing
              │ site
```

FIG. 2

41

Construction of pCH 233

FIG. 3 A

FIG.3 B

```
                100        ←————————— fragment A ————————|—fragment B→
                  *
                                              BstEII    Nco I
                                                ↓         ↓
CCG GCG GCT GCA AAC GCT GAA ACT GCA CAC AAA TCG AAT GAG GTG ACC ATG GAC ....
Pro Ala Ala Ala Asn Ala Glu Thr Ala His Lys Ser Asn Glu Val Thr Met Asp ....
            31                                        41
```

PENAC Sequence

```
                         1                 5                      10
                       Met Lys Asn Arg Asn Arg Met Ile Met Asn Cys
     ◄─────────────────────────────── Pass 1 ──────────────► ◄───────
     1        10           ·20           30              40           50
     AATTCCTAGAGGATATCATT ATG AAA AAT AGA AAT CGT ATG ATC ATG AAC TGT
   ↑   GGATCTCCTATAGTAA TAC TTT TTA TCT TTA GCA TAC TAG TAC TTG ACA
   │       ◄─────────↑──────────────── Pass 2 ───────↑──────────────
   │                 │                                │
   EcoRI           EcoRV                            Bcl I
```

processing site of the PENAC signal sequence

```
                    15                    20                    26      ▼
   Val Thr Ala Ser  Leu Met Tyr Tyr Trp Ser Leu Pro Ala Leu Ala  ▼
   ─────────────────────────── Pass 3 ────────────────────────────►
      60            70            80            90
   GTT ACT GCT TCC CTG ATG TAT TAT TGG AGC TTA CCT GCA CTG G
   CAA TGA CGA AGG GAC TAC ATA ATA ACC TCG AAT GGA CGT GAC CGATC
   ──────────►◄────────── Pass 4 ──────────────────────↑──────►
                                                        │
                                                      Nhe I
```

# FIG. 4

# Construction of pCH 236 and pCH 2361 (pCH 2362)

FIG. 5 A

## Construction of pCH 2363

FIG. 5 B

FIG. 6

FIG. 7

FIG. 8

16.195

FIG. 9

FIG. 10

13.933